# EUROPEAN PATENT APPLICATION

(11) **EP 1 997 799 A1**
(43) Date of publication of application: **03.12.2008**
(21) Application number: 07737502.0
(22) Date of filing: 28.02.2007
(51) Int. Cl.: C07C 211/58, C09K 11/06, H01L 51/50

(54) **AROMATIC AMINE DERIVATIVE AND ORGANIC ELECTROLUMINESCENT DEVICE USING SAME**

(30) Priority: 07.03.2006 JP 2006061313
(71) Applicant: Idemitsu Kosan Co., Ltd., Chiyoda-ku Tokyo 100-8321 (JP)
(72) Inventor: YABUNOUCHI, Nobuhiro, Chiba 299-0293 (JP)
(74) Representative: Gille Hrabal Struck Neidlein Prop Roos
(86) International application number: PCT/JP2007/053748
(87) International publication number: WO 2007/102361

(57) **Abstract**

Provided are a novel aromatic amine derivative represented by the following general formula (1) and an organic electroluminescent device including an organic thin film formed of one or a plurality of layers including at least a light emitting layer interposed between a cathode and an anode, in which at least one layer of the organic thin film contains the aromatic amine derivative alone or as a component of a mixture, which contributes to suppressing molecular crystallization and improving yield in the production of an organic electroluminescent device, whereby an organic electroluminescent device having a long lifetime can be obtained, and the aromatic amine derivative is capable of realizing the organic electroluminescent device: 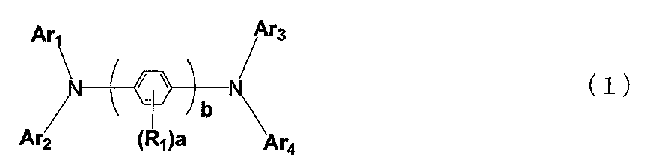 where: R₁ represents an aryl group or the like; a represents an integer of 0 to 4; b represents an integer of 1 to 3; and at least one of Ar₁ to Ar₄ represents a group represented by the following general formula (2): 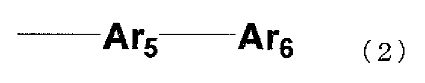 where Ar₅ represents a fused aromatic ring group and Ar₆ represents an aryl group or an aromatic heterocyclic group,
where remaining groups of Ar₁ to Ar₄, none of which is represented by the general formula (2), each independently represent an aryl group or an aromatic heterocyclic group.

## Description

### Technical Field

The present invention relates to an aromatic amine derivative and an organic electroluminescence (EL) device using the same, and more particularly, to an aromatic amine derivative realizing the organic EL device capable of: suppressing the crystallization of a molecule; improving yields upon production of the organic EL device; and increasing a lifetime of the organic EL device by using the aromatic amine derivative having a specific substituent as a hole transporting material.

### Background Art

An organic EL device is a spontaneous light emitting device which utilizes such a principle that a fluorescent substance emits light by virtue of recombination energy of holes injected from an anode and electrons injected from a cathode by an application of an electric field. Since an organic EL device of the laminate type capable of being driven under low electric voltage has been reported by C. W. Tang et al. of Eastman Kodak Company (C. W. Tang and S. A. Vanslyke, Applied Physics Letters, Volume 51, Page 913, 1987, or the like), many studies have been conducted for an organic EL device using an organic material as a constituent material. Tang et al. used tris (8-quinolinolato) aluminum for a light emitting layer and a triphenyldiamine derivative for a hole transporting layer. Advantages of the laminate structure reside in the followings: an efficiency of the hole injection into the light emitting layer can be increased; an efficiency of forming exciton which are formed by blocking and recombining electrons injected from the cathode can be increased; and exciton formed within the light emitting layer can be enclosed. As described above, for the structure of the organic EL device, a two-layered structure having a hole transporting (injecting) layer and an electron transporting emitting layer and a three-layered structure having a hole transporting (injecting) layer, a light emitting layer, an electron transporting (injecting) layer, and the like are widely known. In order to increase the efficiency of recombination of injected holes and electrons in the devices of the laminate type, the device structure and the process for forming the device have been studied.

In general, when an organic EL device is driven or stored in an environment of high temperature, there occur adverse effects such as a change in the luminescent color, a decrease in emission efficiency, an increase in driving voltage, and a decrease in a lifetime of light emission. In order to prevent the adverse effects, it has been necessary that the glass transition temperature (Tg) of the hole transporting material be elevated. Therefore, it is necessary that many aromatic groups be held within a molecule of the hole transporting material (for example, an aromatic diamine derivative of Patent Document 1 and a fused aromatic ring diamine derivative of Patent Document 2), and in general, a structure having 8 to 12 benzene rings is preferably used.
However, when a large number of aromatic groups are present in a molecule, crystallization is liable to occur upon production of the organic EL device through the formation of a thin film by using those hole transporting materials. As a result, there arises a problem such as clogging of an outlet of a crucible to be used in vapor deposition or a reduction in yields of the organic EL device due to generation of defects of the thin film resulting from the crystallization. In addition, a compound having a large number of aromatic groups in any one of its molecules generally has a high glass transition temperature (Tg), but has a high sublimation temperature. Accordingly, there arises a problem in that the lifetime of the compound is short, because a phenomenon such as decomposition at the time of the vapor deposition or the formation of a nonuniform deposition film is expected to occur.
On the other hand, there is a known document disclosing an aromatic diamine derivative in which a fused ring directly bonded to amine contains a substituent. For example, in Patent Document 3, there is described a diamine compound in which a naphthyl group having a methyl group is bonded to amine. However, the inventors of the present invention produced a device using the compound, with a result that the compound had a problem in that the lifetime thereof was short. Further, there is a description on a diamine compound in which a phenyl group is bonded to a naphthyl group, but there is no specific example thereof, and also, there is no description of a feature of substitution with aromatic hydrocarbon at all. Also in Patent Document 4, there is a description on a diamine compound in which a phenyl group is bonded to a naphthyl group, but there is no specific example thereof, and also there is no description of a feature of substitution with aromatic hydrocarbon at all. In Patent Documents 5 and 6, there are reports on an aromatic diamine derivative in which phenanthrene having a substituent is bonded to amine, but a specific description on a compound in which aromatic hydrocarbon is bonded to phenanthrene is not given.
As described above, the organic EL device having a long lifetime has been reported, but it is yet hard to say that the device always shows sufficient performance. In view of the foregoing, development of the organic EL device having a further excellent performance has been strongly desired.
[Patent Document 1] US 4,720,432
[Patent Document 2] US 5,061,569
[Patent Document 3] JP-A-11-149986
[Patent Document 4] JP-A-11-312587
[Patent Document 5] JP-A-11-312586
[Patent Document 6] JP-A-11-135261

### Disclosure of the Invention

### Problems to be solved by the Invention

The present invention has been made with a view to solving the above-mentioned problems, and an object of the present invention is to provide an organic EL device in which a molecule hardly crystallizes, and which can be produced with improved yields and has a long lifetime, and an aromatic amine derivative realizing the organic EL device.

### Means for solving the Problems

The inventors of the present invention have made extensive studies with a view toward achieving the above-mentioned object. As a result, the inventors have found that the above-mentioned problems can be solved by using a novel aromatic amine derivative having a specific substituent represented by the following general formula (1) as a material for an organic EL device, and particularly, as a hole transporting material, and thus the present invention has been completed.
Further, the inventors of the present invention have found that an amino group substituted by an aryl group represented by the general formula (2) is suitable as an amine unit having a specific substituent. The inventors have found that as an interaction between molecules of the amine unit is small because of its steric hindrance, and the unit has such effects that crystallization is suppressed, yield in which an organic EL device is produced is improved, an organic EL device having a long lifetime can be provided, and particularly, a remarkably long lifetime can be attained by combining a blue-based light emitting device.

That is, the present invention provides an aromatic amine derivative represented by the following general formula (1): 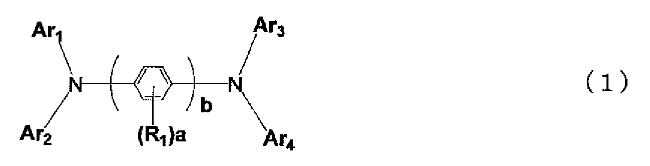 where:
R₁ represents a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen group, a cyano group, a nitro group, a hydroxyl group, or a carboxyl group;
a represents an integer of 0 to 4, and when a represents 2 or more, a plurality of R₁'s may be bonded to each other to form a saturated or unsaturated, five- or six-membered cyclic structure which may be substituted;
b represents an integer of 1 to 3, and when a represents 1 or more and b represents 2 or more, a plurality of R₁'s may be bonded to each other to forma saturated or unsaturated, five-or six-membered cyclic structure which may be substituted; and
at least one of Ar₁ to Ar₄ is a group represented by the following general formula (2): 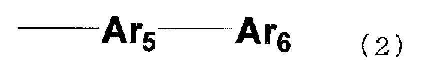
where:
Ar₅ represents a substituted or unsubstituted fused aromatic ring group having 10 to 20 ring carbon atoms; and
Ar₆ represents a substituted or unsubstituted aryl group having 6 to 50 ring carbon atoms or a substituted or unsubstituted aromatic heterocyclic group having 5 to 50 ring carbon atoms,
where remaining groups of Ar₁ to Ar₄, none of which is represented by the general formula (2), each independently represent a substituted or unsubstituted aryl group having 6 to 50 ring carbon atoms or a substituted or unsubstituted aromatic heterocyclic group having 5 to 50 ring carbon atoms.

Further, the present invention provides an organic EL device including an organic thin film layer formed of one or a plurality of layers including at least a light emitting layer interposed between a cathode and an anode, in which at least one layer of the organic thin film layers contains the aromatic amine derivative alone or as a component of a mixture.

### Effect of the Invention

The aromatic amine derivative and the organic EL device using the same of the present invention hardly cause the crystallization of a molecule, and the organic EL device can be produced with improved yields and has a long lifetime. Best Mode for carrying out the Invention

An aromatic amine derivative of the present invention is represented by the following general formula (1): 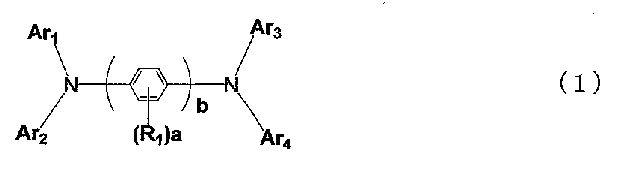 where:
R₁ represents a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen group, a cyano group, a nitro group, a hydroxyl group, or a carboxyl group;
a represents an integer of 0 to 4, and when a represents 2 or more, a plurality of R₁'s may be bonded to each other to form a saturated or unsaturated, five- or six-membered cyclic structure which may be substituted;
b represents an integer of 1 to 3, and when a represents 1 or more and b represents 2 or more, a plurality of R₁'s may be bonded to each other to forma saturated or unsaturated, five- or six-membered cyclic structure which may be substituted; and
at least one of Ar₁ to Ar₄ is a group represented by the following general formula (2): 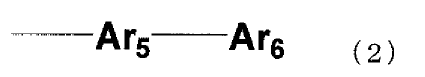
where:
Ar₅ represents a substituted or unsubstituted fused aromatic ring group having 10 to 20 ring carbon atoms; and
Ar₆ represents a substituted or unsubstituted aryl group having 6 to 50 ring carbon atoms or a substituted or unsubstituted aromatic heterocyclic group having 5 to 50 ring carbon atoms,
where remaining groups of Ar₁ to Ar₄, none of which is represented by the general formula (2), each independently represent a substituted or unsubstituted aryl group having 6 to 50 ring carbon atoms or a substituted or unsubstituted aromatic heterocyclic group having 5 to 50 ring carbon atoms.
The aromatic amine derivative according to the present invention represented by the general formula (1) has preferably a total number of carbon atoms excluding substituents of 56 or more and more preferably 68 to 80. In the case where the total number of carbon atoms is less than 56, the glass transition temperature (Tg) decreases, which may cause adverse effects such as a change in the luminescent color, a decrease in emission efficiency, an increase in driving voltage, and a decrease in a lifetime of light emission. On the other hand, in the case where the total number of carbon atoms is more than 80, adverse effects that the sublimation temperature increases, and decomposition at the time of the vapor deposition or the formation of a nonuniform deposition may occur, which may shorten the lifetime.

Examples of the aryl groups of R₁ in the general formula (1) include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl4-yl group, a p-terphenyl 3-yl group, a p-terphenyl 2-yl group, an m-terphenyl 4-yl group, an m-terphenyl 3-yl group, an m-terphenyl 2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-yl group, a fluoranthenyl group, a fluorenyl group, a 1-pyrrolyl group, a 2-pyrrolyl group, a 3-pyrrolyl group, a pyradinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranylgroup, a 3-benzofuranyl group, a 4-benzofuranyl group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrolin-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1,10-phenanthrolin-3-yl group, a 1,10-phenanthrolin-4-yl group, a 1,10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a 1-phenadinyl group, a 2-phenadinyl group, a 1-phenothiadinyl group, a 2-phenothiadinyl group, a 3-phenothiadinyl group, a 4-phenothiadinyl group, a 10-phenothiadinyl group, a 1-phenoxadinyl group, a 2-phenoxadinyl group, a 3-phenoxadinyl group, a 4-phenoxadinyl group, a 10-phenoxadinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrol-1-yl group, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl1-indolyl group, a 4-t-butyl1-indolyl group, a 2-t-butyl3-indolyl group, and a 4-t-butyl3-indolyl group. Of those, a phenyl group, a naphthyl group, a biphenylyl group, an anthranyl group, a phenanthryl group, a pyrenyl group, a chrysenyl group, a fluororanthenyl group, and a fluorenyl group are preferable.

Examples of the alkyl groups of R₁ in the general formula (1) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1, 2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

The alkoxy groups of R₁ in the general formula (1) is represented by -OY, and examples of Y include the same examples as those described for the above-mentioned alkyl group.
Examples of the aralkyl groups of R₁ in the general formula (1) include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, an m-methylbenzyl group, an o-methylbenzyl group, a p-chlorobenzyl group, an m-chlorobenzyl group, an o-chlorobenzyl group, a p-bromobenzyl group, an m-bromobenzyl group, an o-bromobenzyl group, a p-iodobenzyl group, an m-iodobenzyl group, an o-iodobenzyl group, a p-hydroxybenzyl group, an m-hydroxybenzyl group, an o-hydroxybenzyl group, a p-aminobenzyl group, an m-aminobenzyl group, an o-aminobenzyl group, a p-nitrobenzyl group, an m-nitrobenzyl group, an o-nitrobenzyl group, a p-cyanobenzyl group, an m-cyanobenzyl group, an o-cyanobenzyl group, a 1-hydroxy-2-phenylisopropyl group, and a 1-chloro-2-phenylisopropyl, group.

The aryloxy groups of R₁ in the general formula (1) is represented by -OY', and examples of Y' include examples similar to those described for the aryl group.
The arylthio groups of R₁ in the general formula (1) is represented by -SY', and examples of Y' include examples similar to those described for the aryl group.
The alkoxycarbonyl groups of R₁ in the general formula (1) is a group represented by -COOY, and examples of Y include examples similar to those described for the alkyl group.
Examples of an aryl in the amino group substituted by the aryl groups of R₁ in the general formula (1) include examples similar to those described for the aryl group.
Examples of the halogen atoms of R₁ in the general formula (1) include a fluorine atom, a chlorine atom, a bromine atom, and an iodine atom.

In the general formula (1), a represents an integer of 0 to 4, and when a represents 2 or more, a plurality of R₁'s may be bonded to each other to form a saturated or unsaturated, five-or six-membered cyclic structure which may be substituted.
b represents an integer of 1 to 3, and when a represents 1 or more and b represents 2 or more, a plurality of R₁'s may be bonded to each other to forma saturated or unsaturated, five- or six-membered cyclic structure which may be substituted.
Examples of the five- or six-membered cyclic structure which may be formed include: cycloalkanes each having 5 to 12 carbon atoms, such as cyclopentane, cyclohexane, adamantane, and norbornane; cycloalkenes each having 5 to 12 carbon atoms, such as cyclopentene and cyclohexene; cycloalkadienes each having 6 to 12 carbon atoms, such as cyclopentadiene and cyclohexadiene; and aromatic rings each having 6 to 50 carbon atoms, such as benzene, naphthalene, phenanthrene, anthracene, pyrene, chrysene, and acenaphthylene.

The aromatic amine derivative of the present invention has an asymmetric structure when Ar₁ and Ar₂ in the general formula (1) is represented by the general formula (2), and the asymmetric structure is preferable in terms of crystallization suppression or ease of vapor deposition.
The aromatic amine derivative of the present invention is preferable when Ar₁ and Ar₃ in the general formula (1) is represented by the general formula (2), because the production of the aromatic amine derivative becomes easy.
The aromatic amine derivative of the present invention has an asymmetric structure when Ar₁ in the general formula (1) is represented by the general formula (2), and the asymmetric structure is preferable in terms of crystallization suppression or ease of vapor deposition.

Examples of the fused aromatic ring as Ar₅ in the general formula (2) include a divalent residue of naphthalene, phenanthrene, or pyrene, and a preferable example is a divalent residue of naphthalene. Further, examples of a substituted or unsubstituted fused aromatic ring group having 10 to 20 ring carbon atoms as Ar₅ in the general formula (2) include groups in which, of the groups shown as the aryl groups of R₁, the groups other than the phenyl group and the aromatic heterocyclic group are substituted by the arylene groups.
Examples of Ar₆ in the general formula (2) include the same examples as the aryl groups of R₁ in the general formula (1).

The aromatic amine derivative of the present invention is preferable when Ar₅ in the general formula (2) is represented by the general formula (3), because the production of the aromatic amine derivative becomes easy, and in addition, a compound having an excellent solubility can be obtained, whereby purification thereof becomes easy, and an aromatic amine derivative having a high purity can be obtained: 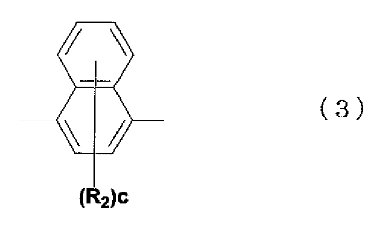 where: R₂ is selected from the same group as that of R₁ in the general formula (1); and c represents an integer of 0 to 6, and when c represents 2 or more, a plurality of R₂'s may be bonded to each other to form a saturated or unsaturated, five- or six-membered cyclic structure which may be substituted.
Examples of each of the substituents of R₂ include the same examples as those of R₁ in the general formula (1). Examples of the five- or six-membered cyclic structure of R₂ include the same examples shown in the general formula (1).
In addition, examples of substituents for Ar₁ to Ar₆ include a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen group, a cyano group, a nitro group, a hydroxyl group, and a carboxyl group. Further, specific examples of an alkyl group, an alkoxy group, an aralkyl group, an aryloxy group, an arylthio group, an alkoxycarbonyl group, and an amino group substituted by an aryl group for Ar₁ to Ar₆ include the same examples as described for R₁.

The aromatic amine derivative of the present invention is preferably represented by the general formula (2), in which Ar₆ is a phenyl group, a biphenyl group, or a naphthyl group.
The aromatic amine derivative of the present invention is preferably represented by the general formula (1), in which Ar₂ is a group represented by the following general formula (4): 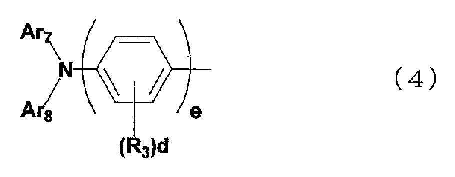 where:
R₃ is selected from the same group as that of R₁ in the general formula (1);
d represents an integer of 0 to 4, and when d represents 2 or more, a plurality of R₃'s may be bonded to each other to form a saturated or unsaturated, five- or six-membered cyclic structure which may be substituted;
e represents an integer of 1 to 3, and when d represents 1 or more and e represents 2 or more, a plurality of R₃'s may be bonded to each other to forma saturated or unsaturated, five- or six-membered cyclic structure which may be substituted; and
Ar₇ and Ar₈ each are a group represented by the general formula (2), or each independently represent a substituted or unsubstituted aryl group having 6 to 50 ring carbon atoms or a substituted or unsubstituted aromatic heterocyclic group having 5 to 50 ring carbon atoms.
In the general formula (4), R₃ is selected from the same groups as those of R₁ in the general formula (1), and the specific examples thereof are also the same as those described on R₁ in the general formula (1).
In the general formula (4), when d represents 2 or more and when d represents 1 or more and e represents 2 or more, a plurality of R₃'s may be bonded to each other to form a saturated or unsaturated, five- or six-membered cyclic structure which may be substituted. Examples of the five- or six-membered cyclic structure which may be formed include: cycloalkanes each having 4 to 12 carbon atoms, such as cyclopentane, cyclohexane, adamantane, and norbornane; cycloalkenes each having 4 to 12 carbon atoms, such as cyclopentene and cyclohexene; cycloalkadienes each having 6 to 12 carbon atoms, such as cyclopentadiene and cyclohexadiene; and aromatic rings each having 6 to 50 carbon atoms, such as benzene, naphthalene, phenanthrene, anthracene, pyrene, chrysene, and acenaphthylene.
In the general formula (4), Ar₇ and Ar₈ each are a group represented by the general formula (2), or each independently represent a substituted or unsubstituted aryl group having 6 to 50 ring carbon atoms or a substituted or unsubstituted aromatic heterocyclic group having 5 to 50 ring carbon atoms, and examples of the substituents thereof include the same substituents as those described for Ar₁ to Ar₆. Further, specific examples of an alkyl group, analkoxygroup, anaralkylgroup, anaryloxygroup, anarylthio group, an alkoxycarbonyl group, and an amino group substituted by an aryl group for Ar₇ and Ar₈ include the same examples as those described for R₁.
The aromatic amine derivative of the present invention is preferably represented by the general formula (1), in which Ar₂ and Ar₄ are each independently a group represented by the general formula (4).

The aromatic amine derivative of the present invention is preferably a material for an organic electroluminescent device.
The aromatic amine derivative of the present invention is preferably a hole transporting material for an organic electroluminescent device.

An organic EL device of the present invention preferably includes an organic thin film layer formed of one or a plurality of layers including at least a light emitting layer interposed between a cathode and an anode, in which at least one layer of the organic thin film layer contains the aromatic amine derivative alone or as a component of a mixture.
In the organic EL device of the present invention, the aromatic amine derivative of the present invention is preferably contained in a hole transporting layer.
Further, in the organic EL device of the present invention, it is preferable that: the organic thin film layer include a hole transporting layer and an electron transporting layer or an electorn injecting layer; the hole transporting layer contain the aromatic amine derivative; and the electron transporting layer or the electorn injecting layer contain a nitrogen-containing heterocyclic compound.
The organic EL device of the present invention preferably emits blue-based light.
The organic EL device of the present invention preferably includes a styrylamine and/or an arylamine in a light emitting layer.

Examples of the aromatic amine derivatives of the present invention, represented by the formula (1), are shown below, but the aromatic amine derivatives are not limited to those exemplified compounds.

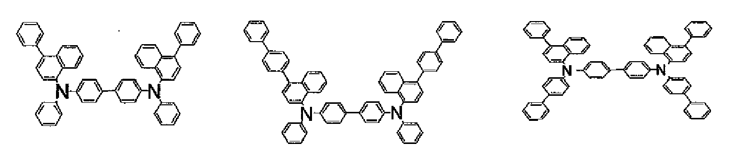

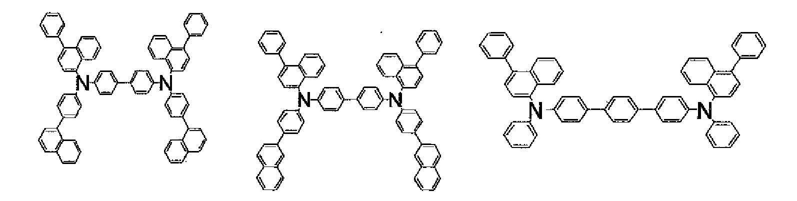

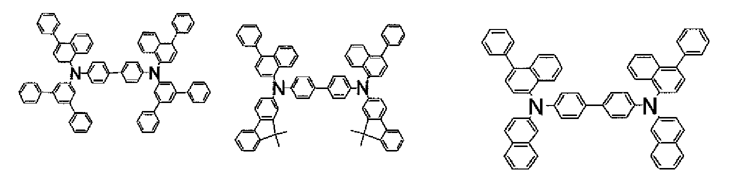

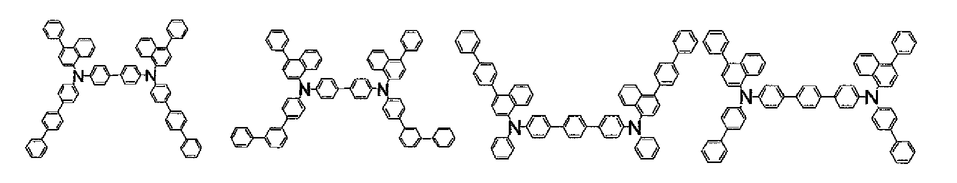

Next, the organic EL device of the present invention is described.
The organic EL device of the present invention includes an organic thin film layer formed of one or a plurality of layers including at least a light emitting layer interposedbetween a cathode and an anode, in which at least one layer of the organic thin film layer contains the aromatic amine derivative alone or as a component of a mixture. In the organic EL device of the present invention, it is preferable that: the organic thin film layers include a hole transporting layer; and the hole transporting layer contain the aromatic amine derivative of the present invention alone or as a component of a mixture. It is more preferable that the hole transporting layer contain the aromatic amine derivative of the present invention as a main component.
The aromatic amine derivative of the present invention is particularly preferably used in an organic EL device that emits blue-based light.

In addition, in the organic EL device of the present invention, the light emitting layer preferably contains an arylamine compound and/or a styrylamine compound, whereby blue-based light is easily obtained.
Examples of the styrylamine compound include compounds each represented by the following general formula (I), and examples of the arylamine compound include compounds each represented by the following general formula (II):

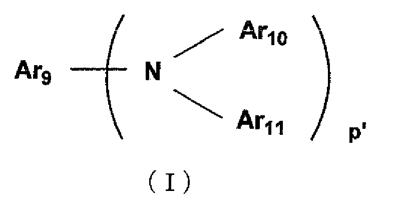

where: Ar₉ represents a group selected from phenyl, biphenyl, terphenyl, stilbene, and distyrylaryl groups; Ar₁₀ and Ar₁₁ each represent a hydrogen atom or an aromatic group having 6 to 20 carbon atoms, and each of Ar₁₀ and Ar₁₁ may be substituted; p' represents an integer of 1 to 4; and Ar₁₀ and/or Ar₁₁ are/is more preferably substituted by styryl groups/a styryl group.
Here, the aromatic group having 6 to 20 carbon atoms is preferably a phenyl group, a naphthyl group, an anthranyl group, a phenanthryl group, a terphenyl group, or the like.

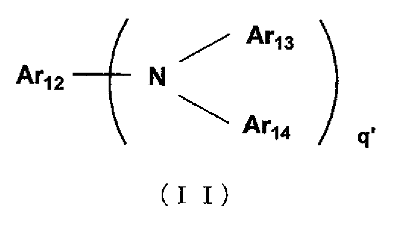

where: Ar₁₂ to Ar₁₄ each represent an aryl group having 5 to 40 ring carbon atoms and which may be substituted; and q' represents an integer of 1 to 4.
Here, examples of the aryl group having 5 to 40 ring atoms preferably include phenyl, naphthyl, anthranyl, phenanthryl, pyrenyl, coronyl, biphenyl, terphenyl, pyrrolyl, furanyl, thiophenyl, benzothiophenyl, oxadiazolyl, diphenylanthranyl, indolyl, carbazolyl, pyridyl, benzoquinolyl, fluoranthenyl, acenaphthofluoranthenyl, and stilbene. In addition, the aryl group having 5 to 40 ring atoms may be further substituted by a substituent. Examples of the substituent preferably include: an alkyl group having 1 to 6 carbon atoms such as an ethyl group, a methyl group, an i-propyl group, an n-propyl group, an s-butyl group, a t-butyl group, a pentyl group, a hexyl group, a cyclopentyl group, or a cyclohexyl group; an alkoxy group having 1 to 6 carbon atoms such as an ethoxy group, a methoxy group, an i-propoxy group, an n-propoxy group, an s-butoxy group, a t-butoxy group, a pentoxy group, a hexyloxy group, a cyclopentoxy group, or a cyclohexyloxy group; an aryl group having 5 to 40 ring atoms; an amino group substituted by an aryl group having 5 to 40 ring atoms; an ester group containing an aryl group having 5 to 40 ring atoms; an ester group containing an alkyl group having 1 to 6 carbon atoms; a cyano group; a nitro group; and a halogen atom such as chlorine, bromine, or iodine.

The structure of the organic EL device of the present invention is described in the following.

### (1) Organic EL device structure

Typical examples of the structure of the organic EL device of the present invention include the following:
(1) an anode/emitting layer/cathode;
(2) an anode/hole injecting layer/emitting layer/cathode;
(3) an anode/emitting layer/electron injecting layer/cathode;
(4) an anode/hole injecting layer/emitting layer/electron injecting layer/cathode;
(5) an anode/organic semiconductor layer/emitting layer/cathode;
(6) an anode/organic semiconductor layer/electron blocking layer/emitting layer/cathode;
(7) an anode/organic semiconductor layer/emitting layer/adhesion improving layer/cathode;
(8) an anode/hole injecting layer/hole transporting layer/emitting layer/electron injecting layer/cathode;
(9) an anode/insulating layer/emitting layer/insulating layer/cathode;
(10) an anode/inorganic semiconductor layer/insulating layer/emitting layer/insulating layer/cathode;
(11) an anode/organic semiconductor layer/insulating layer/emitting layer/insulating layer/cathode;
(12) an anode/insulating layer/hole injecting layer/hole transporting layer/emitting layer/ insulating layer/cathode; and
(13) an anode/insulating layer/hole injecting layer/hole transporting layer/emitting layer/electron injecting layer/cathode.
   Of those, the structure (8) is preferably used in ordinary cases. However, the structure is not limited to the foregoing.
   The aromatic amine derivative of the present invention may be used in any one of the organic thin film layers of the organic EL device. The derivative can be used in a light emitting zone or a hole transporting zone. The derivative is used preferably in the hole transporting zone, or particularly preferably in a hole transporting layer, thereby making a molecule hardly crystallize and improving yields upon production of the organic EL device.
   The amount of the aromatic amine derivative of the present invention to be incorporated into the organic thin film layers is preferably 30 to 100 mol%. In addition, in the case where the aromatic amine derivative of the present invention is incorporated into the hole transporting layer, the amount thereof is preferably 30 to 100 mol%, because when the amount is less than 30 mol%, a hole transporting ability may be decreased.

### (2) Light-transmissive substrate

The organic EL device of the present invention is prepared on a light-transmissive substrate. Here, the light-transmissive substrate is the substrate which supports the organic EL device. It is preferable that the light-transmissive substrate have a transmittance of light of 50% or higher in the visible region of 400 to 700 nm and be flat and smooth.
Examples of the light-transmissive substrate include glass plates and polymer plates. Specific examples of the glass plate include plates formed of soda-lime glass, glass containing barium and strontium, leadglass, aluminosilicate glass, borosilicate glass, barium borosilicate glass, and quartz. Specific examples of the polymer plate include plates formed of polycarbonate, acrylic, polyethylene terephthalate, polyether sulfide, and polysulfone.

### (3) Anode

The anode of the organic EL device of the present invention has the function of injecting holes into the hole transporting layer or the light emitting layer. It is effective that the anode has a work function of 4.5 eV or higher. Specific examples of the material for the anode used in the present invention include indium tin oxide (ITO) alloys, tin oxide (NESA), indium zinc oxide (IZO), gold, silver, platinum, and copper.
The anode can be prepared by forming a thin film of the electrode material described above in accordance with a process such as the vapor deposition process and the sputtering process.
When the light emitted from the light emitting layer is obtained through the anode, it is preferable that the anode have a transmittance of the emitted light higher than 10%. It is also preferable that the sheet resistivity of the anode be several hundred Ω/□ or smaller. The thickness of the anode is, in general, selected in the range of 10 nm to 1 µm and preferably in the range of 10 to 200 nm although the preferable range may be different depending on the used material.

### (4) Emitting layer

The light emitting layer of the organic EL device has a combination of the following functions (1) to (3).
(1) The injecting function: the function of injecting holes from the anode or the hole injecting layer and injecting electrons from the cathode or the electron injecting layer when an electric field is applied.
(2) The transporting function: the function of transporting injected charges (i.e., electrons and holes) by the force of the electric field.
(3) The light emitting function: the function of providing the field for recombination of electrons and holes and leading to the emission of light.
   However, the easiness of injection may be different between holes and electrons and the ability of transportation expressed by the mobility may be different between holes and electrons. It is preferable that either one of the charges be transferred.
   A known method such as a vapor deposition method, a spin coating method, or an LB method is applicable to the formation of the light emitting layer. The light emitting layer is particularly preferably a molecular deposit film. The term "molecular deposit film" as used herein refers to a thin film formed by the deposition of a material compound in a vapor phase state, or a film formed by the solidification of a material compound in a solution state or a liquid phase state. The molecular deposit film can be typically distinguished from a thin film formed by the LB method (molecular accumulation film) on the basis of differences between the films in aggregation structure and higher order structure, and functional differences between the films caused by the foregoing differences.
   In addition, as disclosed in JP-A-57-51781, the light emitting layer can also be formed by: dissolving a binder such as a resin and a material compound in a solvent to prepare a solution; and forming a thin film from the prepared solution by the spin coating method or the like.
   In the present invention, where desired, the light emitting layer may include other known light emitting materials other than the light emittingmaterial composed of the aromatic amine derivative of the present invention, or a light emitting layer including other known light emitting material may be laminated to the light emitting layer including the light emitting material composed of the aromatic amine derivative of the present invention as long as the object of the present invention is not adversely affected.

Examples of a light emitting material or a doping material which can be used in the light emitting layer together with the aromatic amine derivative of the present invention include, but not limited to, anthracene, naphthalene, phenanthrene, pyrene, tetracene, coronene, chrysene, fluoresceine, perylene, phthaloperylene, naphthaloperylene, perynone, phthaloperynone, naphthaloperynone, diphenylbutadiene, tetraphenylbutadiene, coumarin, oxadiazole, aldazine, bisbenzoxazoline, bisstyryl, pyrazine, cyclopentadiene, quinoline metal complexes, aminoquinoline metal complexes, benzoquinoline metal complexes, imine, diphenylethylene, vinylanthracene, diaminocarbazole, pyrane, thiopyrane, polymethine, merocyanine, imidazole-chelated oxynoid compounds, quinacridone, rubrene, and fluorescent dyes.

A host material that can be used in a light emitting layer together with the aromatic amine derivative of the present invention is preferably a compound represented by any one of the following formulae (i) to (ix):
an asymmetric anthracene represented by the following general formula (i): 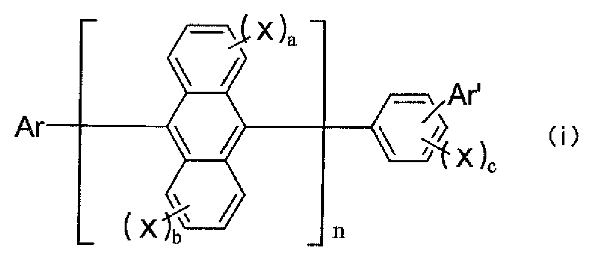
where:
Ar represents a substituted or unsubstituted fused aromatic group having 10 to 50 ring carbon atoms;
Ar' represents a substituted or unsubstituted aromatic group having 6 to 50 ring carbon atoms;
X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group, or a hydroxyl group;
a, b, and c each represent an integer of 0 to 4; and
n represents an integer of 1 to 3, and when n represents 2 or more, anthracene nuclei in [] may be identical to or different from each other;

an asymmetric monoanthracene derivative represented by the following general formula (ii): 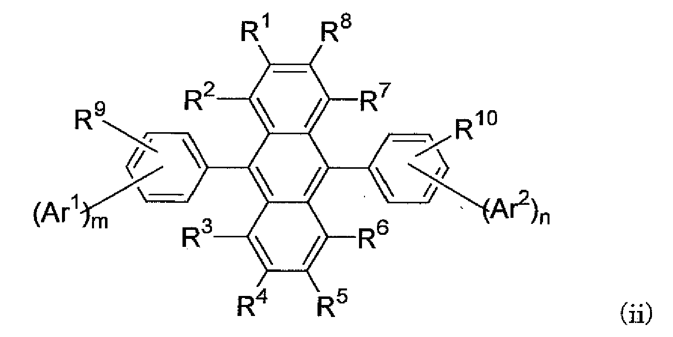 where:
Ar¹ and Ar² each independently represent a substituted or unsubstituted aromatic ring group having 6 to 50 ring carbon atoms; m and n each represent an integer of 1 to 4; provided that Ar¹ and Ar² are not identical to each other when m = n = 1 and positions at which Ar¹ and Ar² are bound to a benzene ring are bilaterally symmetric, and m and n represent different integers when m or n represents an integer of 2 to 4; and
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group;

an asymmetric pyrene derivative represented by the following general formula (iii): 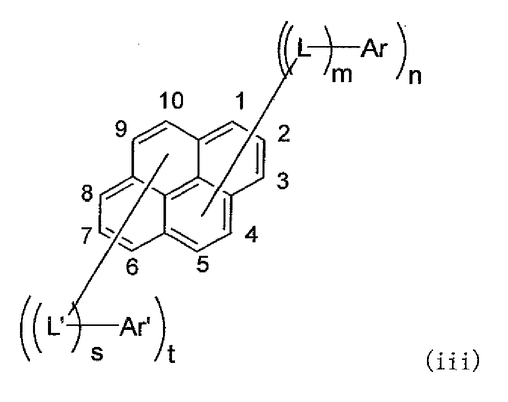 where:
Ar and Ar' each represent a substituted or unsubstituted aromatic group having 6 to 50 ring carbon atoms;
L and L' each represent a substituted or unsubstituted phenylene group, a substituted or unsubstituted naphthalenylene group, a substituted or unsubstituted fluorenylene group, or a substituted or unsubstituted dibenzosilolylene group;
m represents an integer of 0 to 2; n represents an integer of 1 to 4; s represents an integer of 0 to 2; t represents an integer of 0 to 4; and
in addition, L or Ar binds to any one of 1- to 5-positions of pyrene, and L' or Ar' binds to any one of 6- to 10-positions of pyrene,
provided that Ar, Ar', L, and L' satisfy the following item (1) or (2) when n + t represents an even number:
   (1) Art ≠ Ar' and/or L ≠ L' (where the symbol "≠" means that groups connected with the symbol have different structures); and
   (2) when Ar = Ar' and L = L',
      (2-1) m ≠ s and/or n ≠ t, or
      (2-2) when m = s and n = t,
         (2-2-1) in the case, where L and L' (or pyrene) bind (or binds) to different binding positions on Ar and Ar', or (2-2-2) in the case where L and L' (or pyrene) bind (or binds) to the same binding positions on Ar and Ar', the case where the substitution positions of L and L', or of Ar and Ar' in pyrene are 1- and 6-positions, or 2- and 7-positions does not occur;

an asymmetric anthracene derivative represented by the following general formula (iv): 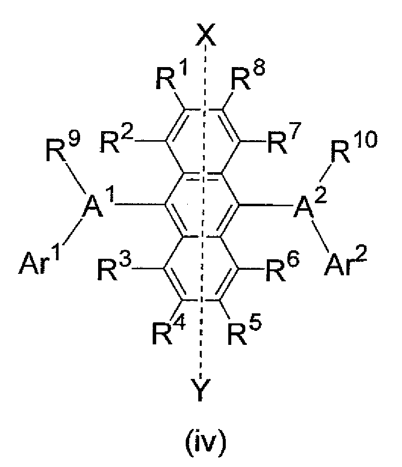 where:
A¹ and A² each independently represent a substituted or unsubstituted fused aromatic ring group having 10 to 20 ring carbon atoms;
Ar¹ and Ar² each independently represent a hydrogen atom, or a substituted or unsubstituted aromatic ring group having 6 to 50 ring carbon atoms;
R¹ to R¹⁰ each independently represent a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group, or a hydroxyl group; and
the number of each of Ar¹, Ar², R⁹, and R¹⁰ may be two or more, and adjacent groups may form a saturated or unsaturated cyclic structure,
provided that the case where groups symmetric with respect to the X-Y axis shown on central anthracene in the general formula (1) bind to 9- and 10-positions of the anthracene does not occur;

an anthracene derivative represented by the following general formula (v): 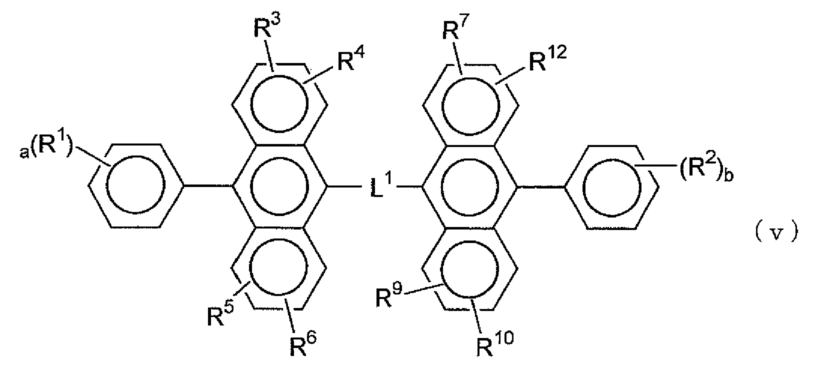 where:
R¹ to R¹⁰ each independently represent a hydrogen atom, an alkyl group, a cycloalkyl group, an aryl group which may be substituted, an alkoxyl group, an aryloxy group, an alkylamino group, an alkenyl group, an arylamino group, or a heterocyclic group which may be substituted; a and b each represent an integer of 1 to 5, and, when a or b represents 2 or more, R¹'s or R²'s may be identical to or different from each other, or R¹'s or R²'s may be bonded to each other to form a ring; R³ and R⁴, R⁵ and R⁵, R⁷ and R⁸, or R⁹ and R¹⁰ may be bonded to each other to form a ring; and L¹ represents a single bond, -O-, -S-, -N(R)- where R represents an alkyl group or an aryl group which may be substituted, an alkylene group, or an arylene group;

an anthracene derivative represented by the following general formula (vi): 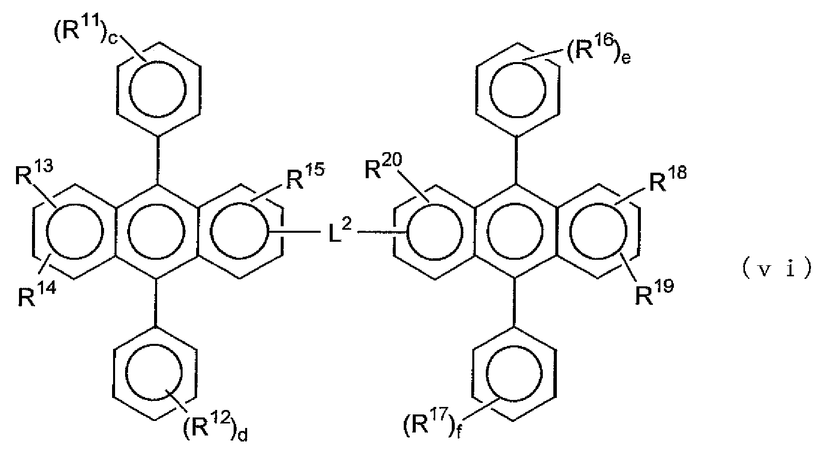 where: R¹¹ to R²⁰ each independently represent a hydrogen atom, an alkyl group, a cycloalkyl group, an aryl group, an alkoxyl group, an aryloxy group, an alkylamino group, an arylamino group, or a heterocyclic group which may be substituted; c, d, e, and f each represent an integer of 1 to 5, and, when any one of c, d, e, and f represents 2 or more, R¹¹'s, R¹²'s, R¹⁶'s, or R¹⁷'s may be identical to or different from each other, or R¹¹'s, R¹²'s, R¹⁶'s, or R¹⁷'s may be bonded to each other to form a ring; R¹³ and R¹⁴, or R¹⁸ and R¹⁹ may be bonded to each other to form a ring; and L² represents a single bond, -O-, -S-, -N (R) - where R represents an alkyl group or an aryl group which may be substituted, an alkylene group, or an arylene group;

a spirofluorene derivative represented by the following general formula (vii): 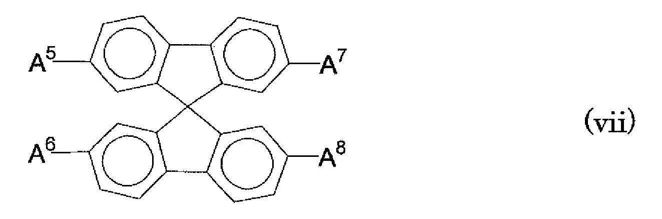 where: A⁵ to A⁸ each independently represent a substituted or unsubstituted biphenyl group, or a substituted or unsubstituted naphthyl group;

a fused ring-containing compound represented by the following general formula (viii): 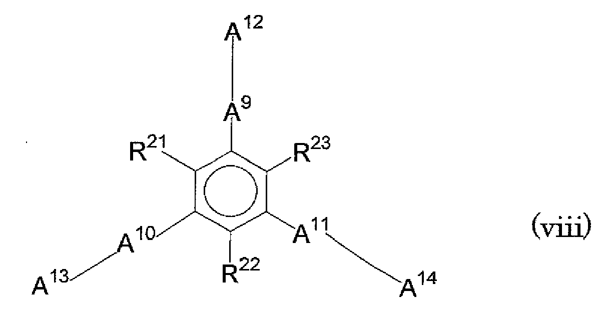 where: A⁹ to A¹⁴ each have the same meaning as that described above; R²¹ to R²³ each independently represent a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 carbon atoms, an aralkyloxy group having 7 to 18 carbon atoms, an arylamino group having 5 to 16 carbon atoms, a nitro group, a cyano group, an ester group having 1 to 6 carbon atoms, or a halogen atom; and at least one of A⁹ to A¹⁴ represents a group having three or more fused aromatic rings; and

a fluorene compound represented by the following general formula (ix): 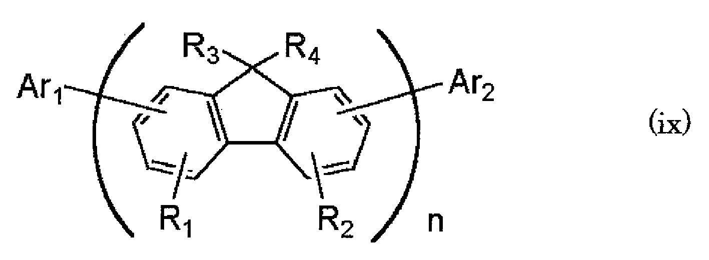 where: R₁ and R₂ each represent a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group, or a halogen atom; R₁'s or R₂'s bonded to different fluorene groups may be identical to or different from each other, and R₁ and R₂ bonded to the same fluorene group may be identical to or different from each other; R₃ and R₄ each represent a hydrogen atom, a substituted or unsubstituted alkyl group, a substituted or unsubstituted aralkyl group, a substituted or unsubstituted aryl group, or a substituted or unsubstituted heterocyclic group; R₃'s or R₄'s bonded to different fluorene groups may be identical to or different from each other, and R₃ and R₄ bonded to the same fluorene group may be identical to or different from each other; Ar₁ and Ar₂ each represent a substituted or unsubstituted fused polycyclic aromatic group having three or more benzene rings in total, or a substituted or unsubstituted fused polycyclic heterocyclic group that has three or more rings each of which is a benzene ring or a heterocyclic ring in total and that is bonded to a fluorene group by carbon, and Ar₁ and Ar₂ may be identical to or different from each other; and n represents an integer of 1 to 10.

Of the above-mentioned host materials, an anthracene derivative is preferable, a monoanthracene derivative is more preferable, and an asymmetric anthracene is particularly preferable.
In addition, a phosphorescent compound can also be used as a dopant light emitting material. A compound containing a carbazole ring as a host material is preferable as the phosphorescent compound. The dopant is a compound capable of emitting light from a triplet exciton, and is not particularly limited as long as light is emitted from a triplet exciton, a metal complex containing at least one metal selected from the group consisting of Ir, Ru, Pd, Pt, Os, and Re is preferable, and a porphyrin metal complex or an orthometalated metal complex is preferable.
A host composed of a compound containing a carbazole ring and suitable for phosphorescence is a compound having a function of causing a phosphorescent compound to emit light as a result of the occurrence of energy transfer from the excited state of the host to the phosphorescent compound. A host compound is not particularly limited as long as it is a compound capable of transferring exciton energy to a phosphorescent compound, and can be appropriately selected in accordance with a purpose. The host compound may have, for example, an arbitrary heterocyclic ring in addition to a carbazole ring.

Specific examples of such a host compound include a carbazole derivative, a triazole derivative, an oxazole derivative, an oxadiazole derivative, an imidazole derivative, a polyarylalkane derivative, a pyrazoline derivative, a pyrazolone derivative, a phenylene diamine derivative, an arylamine derivative, an amino substituted chalcone derivative, a styrylanthracene derivative, a fluorenone derivative, a hydrazone derivative, a stilbene derivative, a silazane derivative, an aromatic tertiary amine compound, a styrylamine compound, an aromatic dimethylidene-based compound, a porphyrin-based compound, an anthraquinodimethane derivative, an anthrone derivative, a diphenylquinone derivative, a thiopyranedioxide derivative, a carbodiimide derivative, a fluorenilidene methane derivative, a distyryl pyrazine derivative, a heterocyclic tetracarboxylic anhydride such as naphthaleneperylene, a phthalocyanine derivative, various metal complex polysilane-based compounds typified by a metal complex of an 8-quinolinol derivative or a metal complex having metal phthalocyanine, benzooxazole, or benzothiazole as a ligand, a poly(N-vinylcarbazole) derivative, an aniline-based copolymer, a conductive high molecular weight oligomer such as a thiophene oligomer or polythiophene, polymer compounds such as a polythiophene derivative, a polyphenylene derivative, a polyphenylene vinylene derivative, and a polyfluorene derivative. One of the host materials may be used alone, or two or more of them may be used in combination.
Specific examples thereof include the compounds as described below.

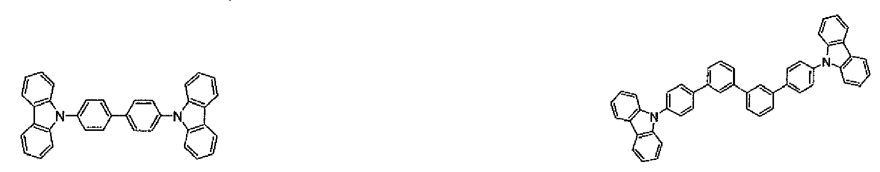

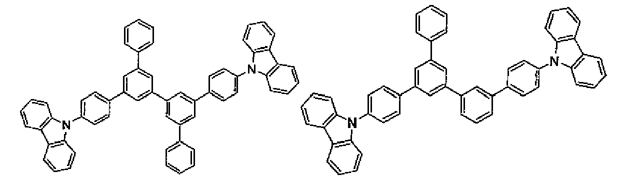

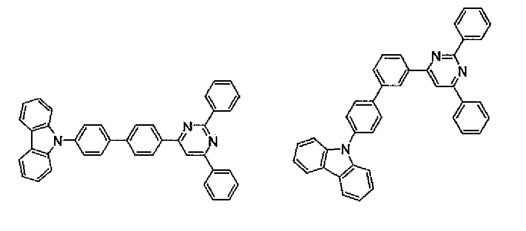

A phosphorescent dopant is a compound capable of emitting light from a triplet exciton. The dopant, which is not particularly limited as long as light is emitted from a triplet exciton, is preferably a metal complex containing at least one metal selected from the group consisting of Ir, Ru, Pd, Pt, Os, and Re, and is preferably a porphyrin metal complex or an orthometalated metal complex. A porphyrin platinum complex is preferable as the porphyrin metal complex. One kind of a phosphorescent compound may be used alone, or two or more kinds of phosphorescent compounds may be used in combination.
Any one of various ligands can be used for forming an orthometalated metal complex. Examples of a preferable ligand include a 2-phenylpyridine derivative, a 7,8-benzoquinoline derivative, a 2-(2-thienyl)pyridine derivative, a 2-(1-naphthyl)pyridine derivative, and a 2-phenylquinoline derivative. Each of those derivatives may have a substituent as required. A fluoride of any one of those derivatives, or one obtained by introducing a trifluoromethyl group into any one of those derivatives is a particularly preferable blue-based dopant. The metal complex may further include a ligand other than the above-mentioned ligands such as acetylacetonato or picric acid as an auxiliary ligand.
The content of the phosphorescent dopant in the light emitting layer is not particularly limited, and can be appropriately selected in accordance with a purpose. The content is, for example, 0.1 to 70 mass%, and is preferably 1 to 30 mass%. When the content of the phosphorescent compound is less than 0.1 mass%, the intensity of emitted light is weak, and an effect of the incorporation of the compound is not sufficiently exerted. When the content exceeds 70 mass%, a phenomenon referred to as concentration quenching becomes remarkable, and device performance reduces.
In addition, the light emitting layer may contain a hole transporting material, an electron transporting material, or a polymer binder as required.
Further, the thickness of the light emitting layer is preferably 5 to 50 nm, more preferably 7 to 50 nm, or most preferably 10 to 50 nm. When the thickness is less than 5 nm, it becomes difficult to form the light emitting layer, so the adjustment of chromaticity may be difficult. When the thickness exceeds 50 nm, the driving voltage may increase.

### (5) Hole injecting and transporting layer (hole transporting zone)

The hole injecting and transporting layer is a layer which helps injection of holes into the light emitting layer and transports the holes to the light emitting region. The layer exhibits a great mobility of holes and, in general, has an ionization energy as small as 5.5 eV or smaller. For such the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.
When the aromatic amine derivative of the present invention is used in the hole transporting zone, the aromatic amine derivative of the present invention may be used alone or as a mixture with other materials for forming the hole injecting and transporting layer.
The material which can be used for forming the hole injecting and transporting layer as a mixture with the aromatic amine derivative of the present invention is not particularly limited as long as the material has a preferable property described above. The material can be arbitrarily selected from materials which are conventionally used as the charge transporting material of holes in photoconductive materials and known materials which are used for the hole injecting and transporting layer in organic EL devices.

Specific examples include: a triazole derivative (see, for example, US 3,112,197); an oxadiazole derivative (see, for example, US 3, 189, 447) ; an imidazole derivative (see, for example, JP-B-37-16096); a polyarylalkane derivative (see, for example, US 3,615,402, US 3, 820, 989, US 3,542,544, JP-B-45-555, JP-B-51-10983, JP-A-51-93224, JP-A-55-17105, JP-A-56-4148, JP-A-55-108667, JP-A-55-156953, and JP-A-56-36656); a pyrazoline derivative and a pyrazolone derivative (see, for example, US 3, 180, 729, US 4, 278, 746, JP-A-55-88064, JP-A-55-88065, JP-A-49-105537, JP-A-55-51086, JP-A-56-80051, JP-A-56-88141, JP-A-57-45545, JP-A-54-112637, and JP-A-55-74546); a phenylenediamine derivative (see, for example, US 3,615,404, JP-B-51-10105, JP-B-46-3712, JP-B-47-25336, JP-A-54-53435, JP-A-54-110536, and JP-A-54-119925); an arylamine derivative (see, for example, US 3,567,450, US 3,180,703, US 3,240,597, US 3,658,520, US 4,232,103, US 4,175,961, US 4,012,376, JP-B-49-35702, JP-B-39-27577, JP-A-55-144250, JP-A-56-119132, JP-A-56-22437, and DE 1,110,518); an amino-substituted chalcone derivative (see, for example, US 3,526,501); an oxazole derivative (those disclosed in US 3,257,203); a styrylanthracene derivative (see, for example, JP-A-56-46234); a fluorenone derivative (see, for example, JP-A-54-110837); a hydrazone derivative (see, for example, US 3,717,462, JP-A-54-59143, JP-A-55-52063, JP-A-55-52064, JP-A-55-46760, JP-A-55-85495, JP-A-57-11350, JP-A-57-148749, and JP-A-2-311591); a stilbene derivative (see, for example, JP-A-61-210363, JP-A-61-228451, JP-A-61-14642, JP-A-61-72255, JP-A-62-47646, JP-A-62-36674, JP-A-62-10652, JP-A-62-30255, JP-A-60-93445, JP-A-60-94462, JP-A-60-174749, and JP-A-60-175052); a silazane derivative (US 4,950,950); a polysilane-based copolymer (JP-A-2-204996); an aniline-based copolymer (JP-A-2-282263); and a conductive high molecular weight oligomer as described in JP-A-1-211399 (in particular, a thiophene oligomer).

In addition to the above-mentioned materials which can be used as the material for the hole injecting and transporting layer, a porphyrin compound (those disclosed in, for example, JP-A-63-2956965); an aromatic tertiary amine compound and a styrylaminecompound (see, for example,US 4,127,412, JP-A-53-27033, JP-A-54-58445, JP-A-54-149634, JP-A-54-64299, JP-A-55-79450, JP-A-55-144250, JP-A-56-119132, JP-A-61-295558, JP-A-61-98353, and JP-A-63-295695) are preferable, and aromatic tertiary amine compounds are particularly preferable.
Further, examples of aromatic tertiary amine compounds include compounds having two fused aromatic rings in the molecule such as 4,4'-bis(N-(1-naphthyl)-N-phenylamino)-biphenyl (hereinafter referred to as NPD) as disclosed in US 5,061,569, and a compound in which three triphenylamine units are bonded together in a star-burst shape, such as 4,4',4"-tris(N-(3-methylphenyl)-N-phenylamino)triphenylamine (hereinafter referred to as MTDATA) as disclosed in JP-A-4-308688.

In addition, examples of the materials which can be used for the hole injecting and transporting layer include acceptor compounds such as a compound represented by the following formula (a) which is disclosed in JP-A-2001-143871 and a compound represented by the following formula (b) which is disclosed in JP-A-2006-503443: 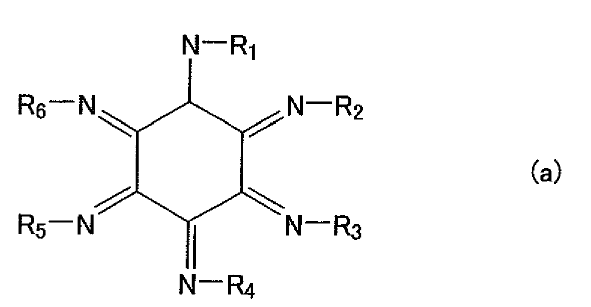 where: R₁ to R₆ each represent one of a substituted or unsubstituted alkyl group, a substituted or unsubstituted aryl group, and a substituted or unsubstituted heterocyclic group, provided that R₁ to R₆ may be identical to or different from each other, and R₁ and R₂, R₃ and R₄, and R₅ and R₆ or R₁ and R₆, R₂ and R₃, and R₄ and R₅ may form a fused ring; and

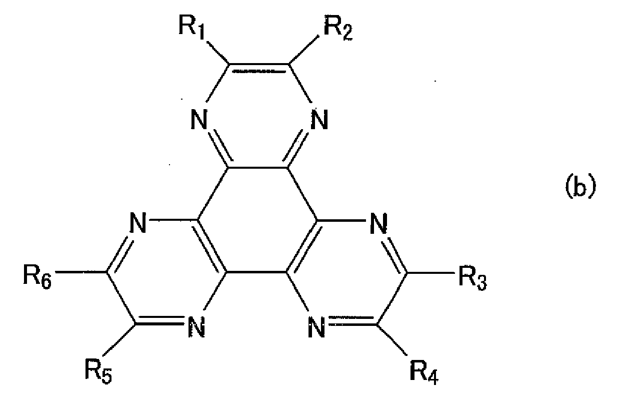

where R₁ to R₆ each represent a substituent, preferably an electrophilic group such as a nitrile group, a nitro group, a sulfonyl group, a trifluoromethyl group, or a halogen.

Further, in addition to the aromatic dimethylidine-based compounds described above as the material for the light emitting layer, inorganic compounds such as Si of the p-type and SiC of the p-type can also be used as the material for the hole injecting and transporting layer.

The hole injecting and transporting layer can be formed by forming a thin layer from the aromatic amine derivative of the present invention in accordance with a known process such as the vacuum vapor deposition process, the spin coating process, the casting process, and the LB process. The thickness of the hole injecting and transporting layer is not particularly limited. In general, the thickness is 5 nm to 5 µm. The hole injecting and transporting layer may be formed of a single layer containing one or more materials described above or may be a laminate formed of hole injecting and transporting layers containing materials different from the materials of the hole injecting and transporting layer described above as long as the aromatic amine derivative of the present invention is incorporated in the hole injecting and transporting zone.
Further, an organic semiconductor layer may be disposed as a layer for helping the injection of holes or electrons into the light emitting layer. As the organic semiconductor layer, a layer having a conductivity of 10⁻¹⁰ S/cm or higher is preferable. As the material for the organic semiconductor layer, oligomers containing thiophene, and conductive oligomers such as oligomers containing arylamine and conductive dendrimers such as dendrimers containing arylamine, which are disclosed in JP-A-8-193191, can be used.

### (6) Electron injecting and transporting layer

Next, the electron injecting and transporting layer is a layer which helps injection of electrons into the light emitting layer, transports the holes to the light emitting region, and exhibits a great mobility of electrons. The adhesion improving layer is an electron injecting layer including a material exhibiting particularly improved adhesion with the cathode.
In addition, it is known that, in an organic EL device, emitted light is reflected by an electrode (cathode in this case), so emitted light directly extracted from an anode and emitted light extracted via the reflection by the electrode interfere with each other. The thickness of an electron transporting layer is appropriately selected from the range of several nanometers to several micrometers in order that the interference effect may be effectively utilized. When the thickness is particularly large, an electron mobility is preferably at least 10⁻⁵ cm²/Vs or more upon application of an electric field of 10⁴ to 10⁶ V/cm in order to avoid an increase in voltage.
A metal complex of 8-hydroxyquinoline or of a derivative of 8-hydroxyquinoline, or an oxadiazole derivative is suitable as a material to be used in an electron injecting layer. Specific examples of the metal complex of 8-hydroxyquinoline or of the derivative of 8-hydroxyquinoline that can be used as an electron injecting material include metal chelate oxynoid compounds each containing a chelate of oxine (generally 8-quinolinol or 8-hydroxyquinoline), such as tris(8-quinolinol)aluminum.

On the other hand, examples of the oxadiazole derivative include electron transfer compounds represented by the following general formula: 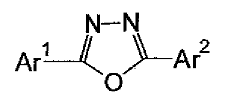 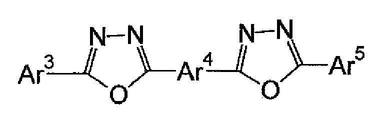 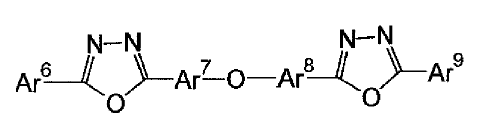 where:Ar¹, Ar², Ar³, Ar⁵, Ar⁶ and Ar⁹ each represent a substituted or unsubstituted aryl group and may represent the same group or different groups; and Ar⁴, Ar⁷ and Ar⁸ each represent a substituted or unsubstituted arylene group and may represent the same group or different groups.
Examples of the aryl group include a phenyl group, a biphenyl group, an anthryl group, a perylenyl group, and a pyrenyl group. Examples of the arylene group include a phenylene group, a naphthylene group, a biphenylene group, an anthranylene group, a perylenylene group, and a pyrenylene group. Examples of the substituent include alkyl groups each having 1 to 10 carbon atoms, alkoxyl groups each having 1 to 10 carbon atoms, and a cyano group. As the electron transfer compound, compounds which can form thin films are preferable.

Examples of the electron transfer compounds described above include the following. 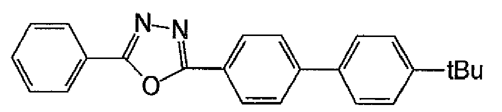 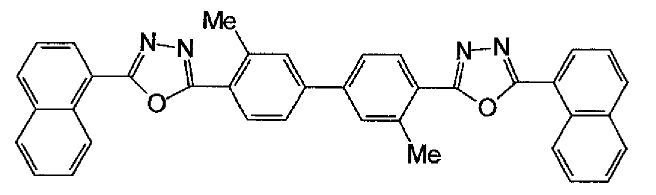 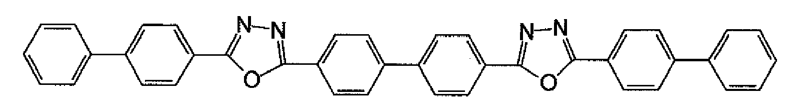 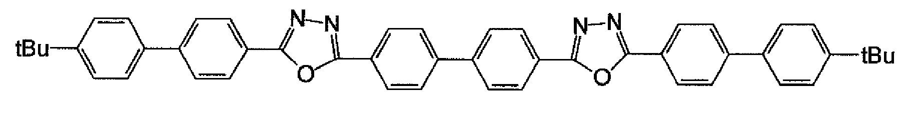 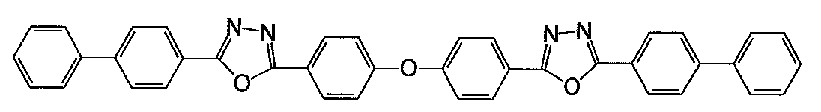 Further, materials represented by the following general formulae (A) to (F) can be used in an electron injecting layer and an electron transporting layer:

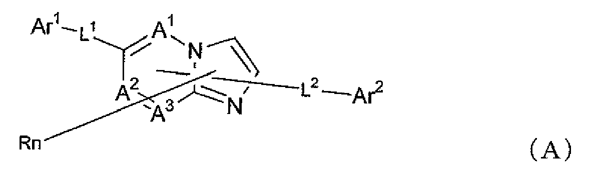

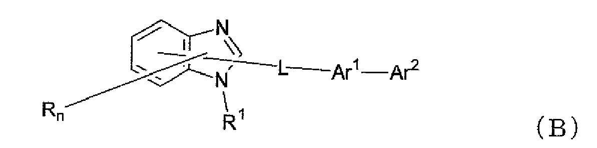

each representing a nitrogen-containing heterocyclic ring derivative, where: A¹ to A³ each independently represent a nitrogen atom or a carbon atom;
Ar¹ represents a substituted or unsubstituted aryl group having 6 to 60 ring carbon atoms, or a substituted or unsubstituted heteroaryl group having 3 to 60 ring carbon atoms, Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms, or a divalent group of any one of them, provided that one of Ar¹ and Ar² represents a substituted or unsubstituted fused ring group having 10 to 60 ring carbon atoms or a substituted or unsubstituted monohetero fused ring group having 3 to 60 ring carbon atoms;
L¹, L², and L each independently represent a single bond, a substituted or unsubstituted arylene group having 6 to 60 ring carbon atoms, a substituted or unsubstituted heteroarylene group having 3 to 60 ring carbon atoms, or a substituted or unsubstituted fluorenylene group; and
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms, n represents an integer of 0 to 5, and, when n represents 2 or more, a plurality of R's may be identical to or different from each other, and a plurality of R groups adjacent to each other may be bonded to each other to form a carbocyclic aliphatic ring or a carbocyclic aromatic ring;

HAr-L-Ar¹-Ar² (C) representing a nitrogen-containing heterocyclic ring derivative, where: HAr represents a nitrogen-containing heterocyclic ring which has 3 to 40 carbon atoms and may have a substituent; L represents a single bond, an arylene group which has 6 to 60 carbon atoms and may have a substituent, a heteroarylene group which has 3 to 60 carbon atoms and may have a substituent, or a fluorenylene group which may have a substituent; Ar¹ represents a divalent aromatic hydrocarbon group which has 6 to 60 carbon atoms and may have a substituent; and Ar² represents an aryl group which has 6 to 60 carbon atoms and may have a substituent, or a heteroaryl group which has 3 to 60 carbon atoms and may have a substituent;

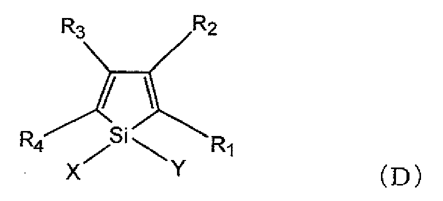

representing a silacyclopentadiene derivative, where:
X and Y each independently represent a saturated or unsaturated hydrocarbon group having 1 to 6 carbon atoms, an alkoxy group, an alkenyloxy group, an alkynyloxy group, a hydroxyl group, a substituted or unsubstituted aryl group, or a substituted or unsubstituted heterocycle, or X and Y are bonded to each other to form a structure as a saturated or unsaturated ring; and R₁ to R₄ each independently represent hydrogen, a halogen atom, a substituted or unsubstituted alkyl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or, when two or more of R₁ to R₄ are adjacent to each other, they form a structure in which a substituted or unsubstituted ring is fused;

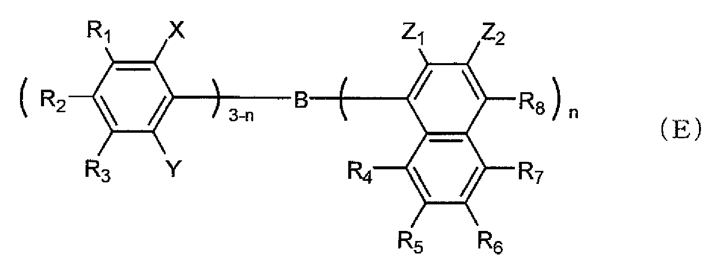

representing a borane derivative, where: R₁ to R₈ and Z₂ each independently represent a hydrogen atom, a saturated or unsaturated hydrocarbon group, an aromatic group, a heterocyclic group, a substituted amino group, a substituted boryl group, an alkoxy group, or an aryloxy group; X, Y, and Z₁ each independently represent a saturated or unsaturated hydrocarbon group, an aromatic group, a heterocyclic group, a substituted amino group, an alkoxy group, or an aryloxy group; substituents of Z₁ and Z₂ may be bonded to each other to form a fused ring; and n represents an integer of 1 to 3, and, when n represents 2 or more, Z₁'s may be different from each other provided that the case where n represents 1, X, Y, and R₂ each represent a methyl group, R₈ represents a hydrogen atom or a substituted boryl group and the case where n represents 3 and Z₁'s each represent a methyl group are excluded; and

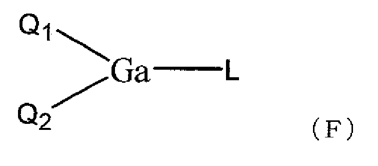

representing a ligand, where: Q¹ and Q² each independently represent a ligand represented by the following general formula (G); and L represents a ligand represented by a halogen atom, a substituted or unsubstituted alkyl group, a substituted or unsubstituted cycloalkyl group, a substituted or unsubstituted aryl group, a substituted or unsubstituted heterocyclic ring group, -OR¹ where R¹ represents a hydrogen atom, a substituted or unsubstituted alkyl group, a substituted or unsubstituted cycloalkyl group, a substituted or unsubstituted aryl group, or a substituted or unsubstituted heterocyclic ring group, or a ligand represented by -O-Ga-Q₃ (Q₄) where Q₃ and Q₄ are identical to Q₁ and Q₂, respectively:

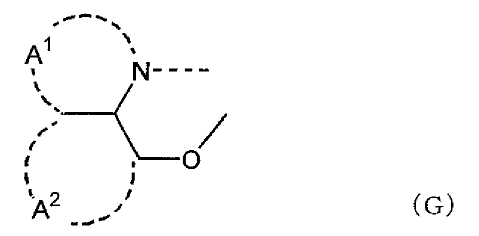

where rings A¹ and A² are six-membered aryl ring structures which are fused with each other and each of which may have a substituent.

The metal complex behaves strongly as an n-type semiconductor, and has a large electron injecting ability. Further, generation energy upon formation of the complex is low. As a result, the metal and the ligand of the formed metal complex are bonded to each other so strongly that the fluorescent quantum efficiency of the complex as a light emitting material improves.
Specific examples of a substituent in the rings A¹ and A² which each form a ligand of the general formula (G) include: a halogen atom such as chlorine, bromine, iodine, or fluorine; a substituted or unsubstituted alkyl group such as a methyl group, an ethyl group, a propyl group, a butyl group, an s-butyl group, a t-butyl group, a pentyl group, a hexyl group, a heptyl group, an octyl group, a stearyl group, or trichloromethyl group; a substituted or unsubstituted aryl group such as a phenyl group, a naphthyl group, a 3-methylphenyl group, a 3-methoxyphenyl group, a 3-fluorophenyl group, a 3-trichloromethylphenyl group, a 3-trifluoromethylphenyl group, or a 3-nitrophenyl group; a substituted or unsubstituted alkoxy group such as a methoxy group, an n-butoxy group, a t-butoxy group, a trichloromethoxy group, a trifluoroethoxy group, a pentafluoropropoxy group, a 2,2,3,3-tetrafluoropropoxy group, an 1,1,1,3,3,3-hexafluoro-2-propoxy group, or a 6-(perfluoroethyl)hexyloxy group; a substituted or unsubstituted aryloxy group such as a phenoxy group, a p-nitrophenoxy group, a p-t-butylphenoxy group, a 3-fluorophenoxy group, a pentafluorophenyl group, or a 3-trifluoromethylphenoxy group; a substituted or unsubstituted alkylthio group such as a methylthio group, an ethylthio group, a t-butylthio group, a hexylthio group, an octylthio group, or a trifluoromethylthio group; a substituted or unsubstituted arylthio group such as a phenylthio group, a p-nitrophenylthio group, a p-t-butylphenylthio group, a 3-fluorophenylthio group, a pentafluorophenylthio group, or a 3-trifluoromethylphenylthio group; a mono-substituted or di-substituted amino group such as a cyano group, a nitro group, an amino group, a methylamino group, a diethylamino group, an ethylamino group, a diethylamino group, a dipropylamino group, a dibutylamino group, or a diphenylamino group; an acylamino group such as a bis (acetoxymethyl) amino group, a bis (acetoxyethyl) amino group, a bis (acetoxypropyl) amino group, or a bis (acetoxybutyl) amino group; a carbamoyl group such as a hydroxyl group, a siloxy group, an acyl group, a methylcarbamoyl group, a dimethylcarbamoyl group, an ethylcarbamoyl group, a diethylcarbamoyl group, a propylcarbamoyl group, a butylcarbamoyl group, or a phenylcarbamoyl group; a cycloalkyl group such as a carboxylic acid group, a sulfonic acid group, an imide group, a cyclopentane group, or a cyclohexyl group; an aryl group such as a phenyl group, a naphthyl group, a biphenylyl group, an anthranyl group, a phenanthryl group, a fluorenyl group, or a pyrenyl group; and a heterocyclic group such as a pyridinyl group, a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triathinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzoimidazolyl group, or a puranyl group. In addition, the above-mentioned substituents may be bound to each other to further form a six-membered aryl ring or a heterocycle.

A preferable embodiment of the organic EL device of the present invention includes an element including a reducing dopant in the region of electron transport or in the interfacial region of the cathode and the organic layer. The reducing dopant is defined as a substance which can reduce a compound having the electron transporting property. Various compounds can be used as the reducing dopant as long as the compounds have a uniform reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.
More specifically, preferable examples of the reducing dopant include substances having a work function of 2.9 eV or smaller, and specific examples of which include at least one alkali metal selected from the group consisting of Na (the work function: 2.36 eV), K (the work function: 2.28 eV), Rb (the work function: 2.16 eV), and Cs (the work function: 1.95 eV) and at least one alkaline earth metal selected from the group consisting of Ca (the work function: 2.9 eV), Sr (the work function: 2.0 to 2.5 eV), and Ba (the work function: 2.52 eV). Of those, at least one alkali metal selected from the group consisting of K, Rb, and Cs is more preferable, Rb and Cs are still more preferable, and Cs is most preferable as the reducing dopant. In particular, those alkali metals have great reducing ability, and the luminance of the emitted light and the lifetime of the organic EL device can be increased by addition of a relatively small amount of the alkali metal into the electron injecting zone. As the reducing dopant having a work function of 2.9 eV or smaller, combinations of two or more alkali metals thereof are also preferable. Combinations having Cs such as the combinations of Cs and Na, Cs and K, Cs and Rb, and Cs, Na, and K are particularly preferable. The reducing ability can be efficiently exhibited by the combination having Cs. The luminance of emitted light and the lifetime of the organic EL device can be increased by adding the combination having Cs into the electron injecting zone.

The present invention may further include an electron injecting layer which is composed of an insulating material or a semiconductor and disposedbetween the cathode and the organic layer. At this time, the electron injecting property can be improved by preventing a leak of electric current effectively. As the insulating material, at least one metal compound selected from the group consisting of alkali metal chalcogenides, alkaline earth metal chalcogenides, alkali metal halides, and alkaline earth metal halides is preferable. It is preferable that the electron injecting layer be composed of the above-mentioned substance such as the alkali metal chalcogenide since the electron injecting property can be further improved. Preferable examples of the alkali metal chalcogenide include Li₂O, K₂O, Na₂S, Na₂Se, and Na₂O. To be specific, preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such as CaF₂, BaF₂, SrF₂, MgF₂, and BeF₂ and halides other than the fluorides.
Examples of the semiconductor composing the electron transporting layer include oxides, nitrides, and oxide nitrides of at least one element selected from Ba, Ca, Sr, Yb, Al, Ga, In, Li, Na, Cd, Mg, Si, Ta, Sb, and Zn used alone or in combination of two or more. It is preferable that the inorganic compound composing the electron transporting layer form a crystallite or amorphous insulating thin film. When the electron transporting layer is composed of the insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

(7) Cathode
As the cathode, a material such as a metal, an alloy, an electroconductive compound, or a mixture of those materials which has a small work function (4 eV or smaller) is used because the cathode is used for injecting electrons to the electron injecting and transporting layer or the light emitting layer. Specific examples of the electrode material include sodium, sodium-potassium alloys, magnesium, lithium, magnesium-silver alloys, aluminum/aluminum oxide, aluminum-lithium alloys, indium, and rare earth metals.
The cathode can be prepared by forming a thin film of the electrode material described above in accordance with a process such as the vapor deposition process or the sputtering process.
When the light emitted from the light emitting layer is obtained through the cathode, it is preferable that the cathode have a transmittance of the emitted light higher than 10%.
It is also preferable that the sheet resistivity of the cathode be several hundred Ω/□ or smaller. The thickness of the cathode is, in general, selected in the range of 10 nm to 1 µm and preferably in the range of 50 to 200 nm.

(8) Insulating layer Defects in pixels tend to be formed in organic EL device due to leak and short circuit since an electric field is applied to ultra-thin films. In order to prevent the formation of the defects, a layer of a thin film having an insulating property may be inserted between the pair of electrodes.
Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned compounds may also be used.

(9) Method of producing the organic EL device
In order to prepare the organic EL device of the present invention, the anode and the light emitting layer, and, where necessary, the hole injecting and transporting layer and the electron injecting and transporting layer are formed in accordance with the illustrated process using the illustrated materials, and the cathode is formed in the last step. The organic EL device may also be prepared by forming the above-mentioned layers in the order reverse to the order described above, i.e., the cathode being formed in the first step and the anode in the last step.
Hereinafter, an embodiment of the process for preparing an organic EL device having a construction in which an anode, a hole injecting layer, a light emitting layer, an electron injecting layer, and a cathode are disposed successively on a light-transmissive substrate will be described.
On a suitable light-transmissive substrate, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process, or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions be suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C; and the thickness of the film: 5 nm to 5 µm although the conditions of the vacuum vapor deposition are different depending on the compound to be used (i.e., material for the hole injecting layer) and the crystal structure and the recombination structure of the target hole injecting layer.

Then, the light emitting layer is formed on the hole injecting layer formed above. A thin film of the organic light emitting material can be formed by using a desired organic light emitting material in accordance with a process such as the vacuum vapor deposition process, the sputtering process, the spin coating process, or the casting process, and the formed thin film is used as the light emitting layer. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor depositionprocess canbe selected in the same ranges as the conditions described for the vacuum vapor deposition of the hole injecting layer, although the conditions are different depending on the compound to be used.
Next, an electron injecting layer is formed on the light emitting layer formed above. Similarly to the hole injecting layer and the light emitting layer, it is preferable that the electron injecting layer be formed in accordance with the vacuum vapor deposition process since a uniform film must be obtained. The conditions of the vacuum vapor deposition can be selected in the same ranges as the condition described for the vacuum vapor deposition of the hole injecting layer and the light emitting layer.
When the vapor deposition process is used, the aromatic amine derivative of the present invention can be deposited by vapor in combination with other materials, although the situation may be different depending on which layer in the light emitting zone or in the hole transporting zone includes the compound. When the spin coating process is used, the compound can be incorporated into the formed layer by using a mixture of the compound with other materials.
A cathode is laminated in the last step, and an organic EL device can be obtained.
The cathode is formed of a metal and can be formed in accordance with the vacuum vapor deposition process or the sputtering process. It is preferable that the vacuum vapor deposition process be used in order to prevent formation of damages on the lower organic layers during the formation of the film.
In the above-mentioned preparation of the organic EL device, it is preferable that the above-mentioned layers from the anode to the cathode be formed successively while the preparation system is kept in a vacuum after being evacuated once.

The method of forming the layers in the organic EL device of the present invention is not particularly limited. A conventionally known process such as the vacuum vapor deposition process or the spin coating process can be used. The organic thin film layer which is used in the organic EL device of the present invention and includes the compound represented by general formula (1) described above can be formed in accordance with a known process such as the vacuum vapor deposition process or the molecular beam epitaxy process (MBE process) or, using a solution prepared by dissolving the compounds into a solvent, in accordance with a coating process such as the dipping process, the spin coating process, the casting process, the bar coating process, or the roll coating process.
The thickness of each layer in the organic thin film layer in the organic EL device of the present invention is not particularly limited. In general, an excessively thin layer tends to have defects such as pin holes, whereas an excessively thick layer requires a high applied voltage to decrease the efficiency. Therefore, a thickness in the range of several nanometers to 1 µm is preferable.
The organic EL device which can be prepared as described above emits light when a direct voltage of 5 to 40 V is applied in the condition that the polarity of the anode is positive (+) and the polarity of the cathode is negative (-). When the polarity is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

Hereinafter, the present invention is described in more detail on the basis of Synthesis Examples and Examples.

### Synthesis Example 1 (synthesis of Intermediate 1)

Under an argon current, to a three-necked flask, 81.9 g of phenylboronic acid, 200 g of dibromonaphthalene, 16.2 g of tetrakis (triphenylphosphine) palladium (Pd(PPh₃)₄), 1,050 ml of 2M Na₂CO₃ solution, 3.4 L of dimethoxyethane, and 3L of toluene were charged, and the mixture was refluxed for 8 hours. The reactant was extracted with toluene and water, followed by drying with anhydrous sodium sulfate.
The resultant was concentrated under reduced pressure, and the obtained crude product was subjected to column purification, whereby the following Intermediate 1 was obtained as 70 g of white powder. By an FD-MS (field desorption mass spectrometry) analysis, main peaks of m/z = 282 and m/z = 284 with respect to C₁₆H₁₁Br = 283 were obtained, so the white powder was identified as the following Intermediate 1.

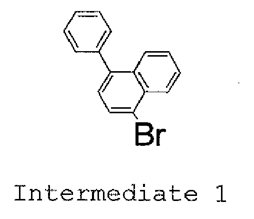

### Synthesis Example 2 (synthesis of Intermediate 2)

The same reaction as in Synthesis Example 1 was carried out except that 138.5 g of 4-biphenylboronic acid was used instead of 81.9 g of phenylboronic acid, whereby the following Intermediate 2 was obtained as 90 g of white powder. By an FD-MS analysis, main peaks of m/z = 358 and m/z = 360 with respect to C₂₂H₁₅Br = 359 were obtained, so the white powder was identified as the following Intermediate 2.

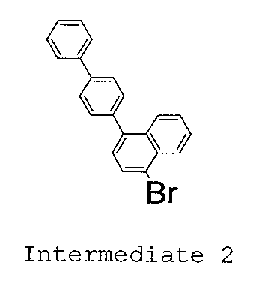

Under an argon current, to a 500-ml three-necked flask, 9.7 g of 1-naphthylboronic acid (manufactured by Aldrich Chemical Company, Inc.), 13.3 g of 4-iodobrombenzene, 1.9 g of tetrakis(triphenylphosphine) palladium (Pd(PPh₃)₄), 50 ml of 2M Na₂CO₃ solution, and 80 ml of dimethoxyethane were charged, and the mixture was refluxed for 8 hours. The reactant was extracted with toluene and water, followed by drying with anhydrous sodium sulfate. The resultant was concentrated under reduced pressure, and the obtained crude product was subj ected to column purification, whereby the following Intermediate 3 was obtained as 8.8 g of white powder. By an FD-MS analysis, main peaks of m/z = 282 and m/z = 284 with respect to C₁₆H₁₁Br = 283 were obtained, so the white powder was identified as the following Intermediate 3.

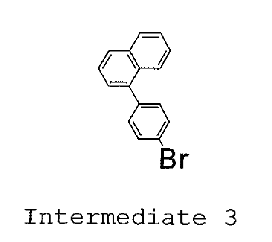

### Synthesis Example 4 (synthesis of Intermediate 4)

The same reaction as in Synthesis Example 22 was carried out except that 9.7 g of 2-naphthylboronic acid (manufactured by Aldrich Chemical Company, Inc.) was used insteadof 9.7 g of 1-naphthylboronic acid, whereby the following Intermediate 4 was obtained as 7.6 g of white powder. By an FD-MS analysis, a main peak of m/z = 284 with respect to C₁₆H₁₁Br = 283 was obtained, so the white powder was identified as the following Intermediate 4.

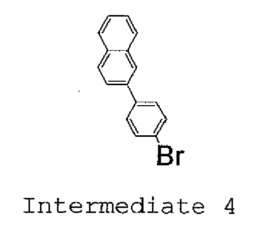

### Synthesis Example 5 (synthesis of Intermediate 5)

Under an argon current, to a 300-ml three-necked flask, 25.0 g of 1-amino-4-bromonaphthalene, 16. 5 g of phenylboronic acid, 1. 04 g of tris(dibenzylideneacetone)dipalladium(0) (manufactured by Aldrich Chemical Company, Inc.), 19.7 g of potassium fluoride, 593 mg of tri-t-butylphosphine, and 100 ml of dehydrated tetrahydrofuran were charged, and the resultant mixture was allowed to react at 80°C for 8 hours.
After the reactant was cooled, 500 ml of water was added to the reactant, and the mixture was filtered with celite. The filtrate was extracted with toluene and dried with anhydrous magnesium sulfate. The resultant was concentrated under reduced pressure, and the obtained crude product was subjected to column purification. The purified product was recrystallized with toluene, followed by filtration and drying, whereby 13.2 g of pale yellow powder was obtained. By an FD-MS analysis, a main peak of m/z = 219 with respect to C₁₆H₁₃N = 219 was obtained, so the pale yellow powder was identified as the following Intermediate 5.

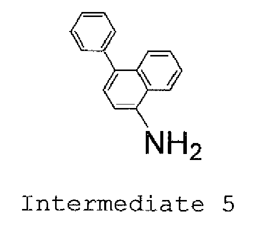

### Synthesis Example 6 (synthesis of Intermediate 6)

Under an argon current, 21.9 g of Intermediate 5, 23.4 g of 4-bromobiphenyl, 13.4 g of sodium t-butoxide (manufactured by Hiroshima Wako Co., Ltd.), 0.92 g of tris(dibenzylideneacetone)dipalladium(0) (manufactured by Aldrich Chemical Company, Inc.), 400 mg of tri-t-butylphosphine, and 300 ml of dehydrated toluene were charged, and the resultant mixture was allowed to react at 80°C for 8 hours.
After the reactant was cooled, 500 ml of water was added to the reactant, and the mixture was filtered with celite. The filtrate was extracted with toluene and dried with anhydrous magnesium sulfate. The resultant was concentrated under reduced pressure, and the obtained crude product was subjected to column purification. The purified product was recrystallized with toluene, followed by filtration and drying, whereby 21.2 g of pale yellow powder was obtained. By an FD-MS analysis, a main peak of m/z = 371 with respect to C₂₈H₂₁N = 371 was obtained, so the pale yellow powder was identified as the following Intermediate 6.

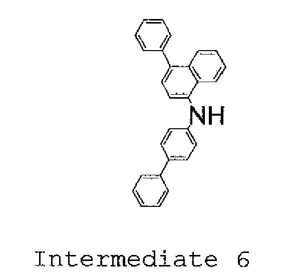

### Synthesis Example 7 (synthesis of Intermediate 7)

The same reaction as in Synthesis Example 6 was carried out except that 28.2 g of Intermediate 3 was used instead of 23.4 g of 4-bromobiphenyl, whereby the following Intermediate 7 was obtained as 28.5 g of white powder. By an FD-MS analysis, a main peak of m/z = 421 with respect to C₃₂H₂₃N = 421 was obtained, so the white powder was identified as the following Intermediate 7.

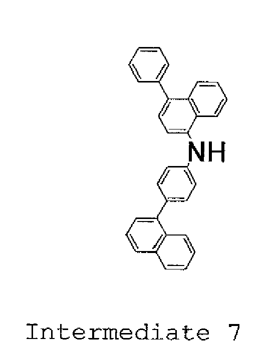

### Synthesis Example 8 (synthesis of Intermediate 8)

The same reaction as in Synthesis Example 6 was carried out except that 28.2 g of Intermediate 4 was used instead of 23.4 g of 4-bromobiphenyl, whereby the following Intermediate 8 was obtained as 23.1 g of whitish yellow powder. By an FD-MS analysis, a main peak of m/z = 421 with respect to C₃₂H₂₃N = 421 was obtained, so the whitish yellow powder was identified as the following Intermediate 8.

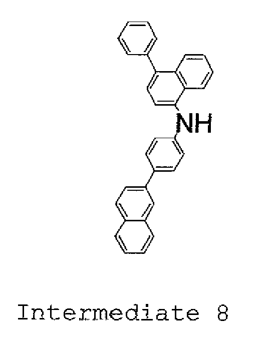

### Synthesis Example 9 (synthesis of Intermediate 9)

The same reaction as in Synthesis Example 6 was carried out except that 15.7 g of 4-bromobenzene was used instead of 23.4 g of 4-bromobiphenyl, whereby the following Intermediate 9 was obtained as 21.1 g of white powder. By an FD-MS analysis, a main peak of m/z = 295 with respect to C₂₂H₇N = 295 was obtained, so the white powder was identified as the following Intermediate 9.

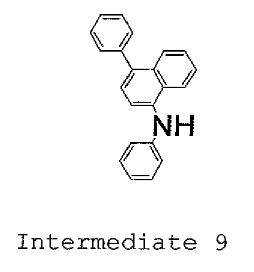

### Synthesis Embodiment 1 (synthesis of Compound H1)

Under an argon current, 16.7 g of N,N'-diphenylbenzidine, 28.2 g of Intermediate 1, 13.4 g of sodium t-butoxide (manufactured by Hiroshima Wako Co., Ltd.), 0.92 g of tris(dibenzylideneacetone)dipalladium(0) (manufactured by Aldrich Chemical Company, Inc.), 400 mg of tri-t-butylphosphine, and 300 ml of dehydrated toluene were charged, and the resultant mixture was allowed to react at 80°C for 8 hours.
After the reactant was cooled, 500 ml of water was added to the reactant, and the mixture was filtered with celite. The filtrate was extracted with toluene and dried with anhydrous magnesium sulfate. The resultant was concentrated under reduced pressure, and the obtained crude product was subjected to column purification. The purified product was recrystallized with toluene, followed by filtration and drying, whereby 26.7 g of pale yellow powder was obtained. By an FD-MS analysis, a main peak of m/z = 740 with respect to C₅₆H₄₀N₂= 740 was obtained, so the pale yellow powder was identified as the following Compound H1.

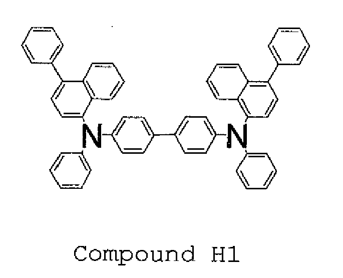

### Synthesis Embodiment 2 (synthesis of Compound H2)

The same reaction as in Synthesis Embodiment 1 was carried out except that 35.8 g of Intermediate 2 was used instead of Intermediate 1, whereby 31.4 g of pale yellow powder was obtained. By an FD-MS analysis, a main peak of m/z = 892 with respect to C₆₈H₄₈N₂ = 892 was obtained, so the pale yellow powder was identified as the following Compound H2.

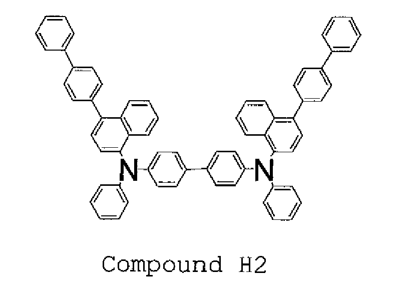

### Synthesis Embodiment 3 (synthesis of Compound H3)

Under an argon current, 20.3 g of 4-4'-diiodobiphenyl, 37.1 g of Intermediate 6, 13.4 g of sodium t-butoxide (manufactured by Hiroshima Wako Co., Ltd.), 0.92 g of tris(dibenzylideneacetone)dipalladium(0) (manufactured by Aldrich Chemical Company, Inc.), 400 mg of tri-t-butylphosphine, and 300 ml of dehydrated toluene were charged, and the resultant mixture was allowed to react at 80°C for 8 hours.
After the reactant was cooled, 500 ml of water was added to the reactant, and the mixture was filtered with celite. The filtrate was extracted with toluene and dried with anhydrous magnesium sulfate. The resultant was concentrated under reduced pressure, and the obtained crude product was subjected to column purification. The purified product was recrystallized with toluene, followed by filtration and drying, whereby 22.9 g of pale yellow powder was obtained. By an FD-MS analysis, a main peak of m/z = 892 with respect to C₆₃H₄₈N₂ = 892 was obtained, so the pale yellow powder was identified as the following Compound H3.

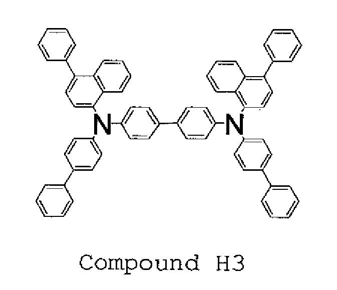

### Synthesis Embodiment 4 (synthesis of Compound H4)

The same reaction as in Synthesis Embodiment 3 was carried out except that 42.1 g of Intermediate 7 was used instead of Intermediate 6, whereby 30.4 g of pale yellow powder was obtained. By an FD-MS analysis, a main peak of m/z = 992 with respect to C₇₆H₅₂N₂ = 992 was obtained, so the pale yellow powder was identified as the following Compound H4.

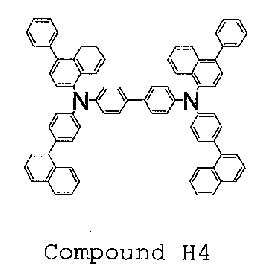

### Synthesis Embodiment 5 (synthesis of Compound H5)

The same reaction as in Synthesis Embodiment 3 was carried out except that 42.1 g of Intermediate 8 was used instead of Intermediate 6, whereby 38.9 g of pale yellow powder was obtained. By an FD-MS analysis, a main peak of m/z = 992 with respect to C₇₆H₅₂N₂ = 992 was obtained, so the pale yellow powder was identified as the following Compound H5.

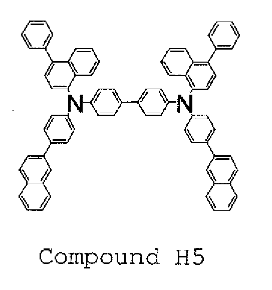

### Synthesis Embodiment 6 (synthesis of Compound H6)

The same reaction as in Synthesis Embodiment 3 was carried out except that 24.1 g of 4-4"-diiodo-p-terphenyl was used instead of 20.3 g of 4-4'-diiodobiphenyl and 29.5 g of Intermediate 9 was used instead of Intermediate 6, whereby 38.9 g of pale yellow powder was obtained. By an FD-MS analysis, a main peak of m/z = 816 with respect to C₆₂H₄₄N₂ = 816 was obtained, so the pale yellow powder was identified as the following Compound H6.

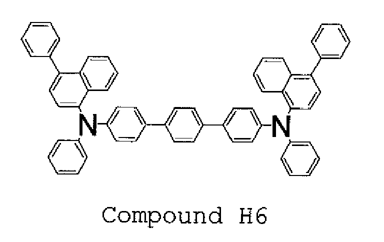

### Example 1 (production of organic EL device)

A glass substrate with an ITO transparent electrode measuring 25 mm wide by 75 mm long by 1.1 mm thick (manufactured by GEOMATEC Co., Ltd.) was subjected to ultrasonic cleaning in isopropyl alcohol for 5 minutes. After that, the substrate was subjected to UV ozone cleaning for 30 minutes.
The glass substrate with the transparent electrode line after the washing was mounted on a substrate holder of a vacuum deposition device. First, Compound H232 to be described below was formed into a film having a thickness of 60 nm on the surface on the side where the transparent electrode line was formed to cover the transparent electrode. The H232 film functions as a hole injecting layer. Compound H1 layer described above was formed into a film having a thickness of 20 nm as the hole transporting material on the H232 film. The film functions as a hole transporting layer. Further, Compound EM1 to be described below was deposited from the vapor and formed into a film having a thickness of 40 nm. Simultaneously with this formation, Amine Compound D1 having a styryl group to be described below, as a light emitting molecule, was deposited from the vapor in such a manner that a weight ratio between Compound EM1 and Amine Compound D1 would be 40:2. The film functions as a light emitting layer.
Alq to be described below was formed into a film having a thickness of 10 nm on the resultant film. The film functions as an electron injecting layer. After that, Li serving as a reducing dopant (Li source: manufactured by SAES Getters) and Alq were subjected to co-deposition. Thus, an Alq:Li film (having a thickness of 10 nm) was formed as an electron injecting layer (cathode). Metal Al was deposited from the vapor onto the Alq:Li film to form a metal cathode. Thus, an organic EL device was formed.
In addition, the luminous efficiency of the resultant organic EL device was measured, and the luminescent color of the device was observed. A luminous efficiency at a current density of 10 mA/cm² was calculated by measuring a luminance by us ing a CS 1000 manufactured by Minolta. Further, the half lifetime of light emission in DC constant current driving at an initial luminance of 5,000 nits and room temperature was measured. Table 1 shows the results thereof.

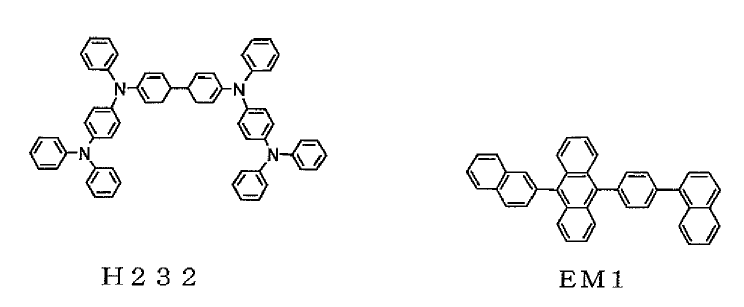

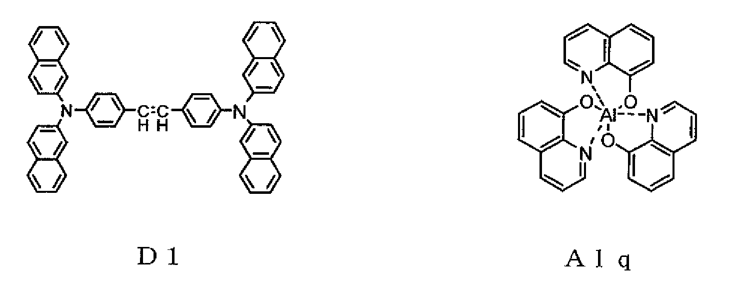

### Examples 2 to 6 (production of organic EL device)

Each organic EL device was produced in the same manner as in Example 1 except that the compound shown in Table 1 was used as a hole transporting material instead of Compound H1. The luminous efficiency of the resultant organic EL device was measured, and the luminescent color of the device was observed.
Further, the half lifetime of light emission in DC constant current driving at an initial luminance of 5,000 nits and room temperature was measured. Table 1 shows the results thereof.

### Comparative Example 1

An organic EL device was produced in the same manner as in Example 1 except that Comparative Compound 1 (Comparative Example 1) was used as a hole transporting material instead of Compound H1. Comparative Compound 1 was crystallized during vapor deposition, whereby a normal device was not produced.
In addition, the luminous efficiency of the resultant organic EL device was measured, and the luminescent color of the device was observed. Further, the half lifetime of light emission in DC constant current driving at an initial luminance of 5, 000 nits and room temperature was measured. Table 1 shows the results thereof.

### Comparative Example 2 (production of organic EL device)

An organic EL device was produced in the same manner as in Example 1 except that Comparative Compound 2 was used as a hole transporting material instead of Compound H1.
The luminous efficiency of the resultant organic EL device was measured, and the luminescent color of the device was observed. Further, the half lifetime of light emission in DC constant current driving at an initial luminance of 5,000 nits and room temperature was measured. Table 1 shows the results thereof.

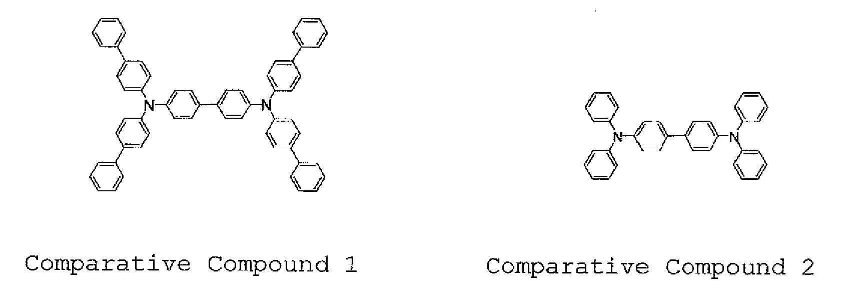

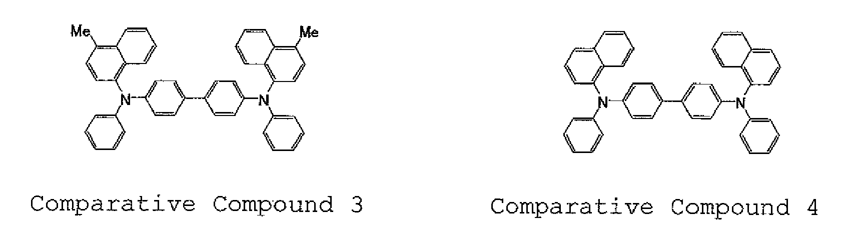

**Table 1**

| Example | Hole transporting material | Luminous efficiency (cd/A) | Luminescent color | Half lifetime (hours) |
|---|---|---|---|---|
| 1 | H1 | 5.1 | Blue | 390 |
| 2 | H2 | 5.1 | Blue | 440 |
| 3 | H3 | 4.9 | Blue | 410 |
| 4 | H4 | 5 | Blue | 400 |
| 5 | H5 | 5.1 | Blue | 410 |
| 6 | H6 | 5 | Blue | 320 |
| Comparative Example 1 | Comparative Compound 1 | 5.1 | Blue | 280 |
| Comparative Example 2 | Comparative Compound 2 | 4.9 | Blue | 170 |
| Comparative Example 4 | Comparative Compound 3 | 4.7 | Blue | 110 |
| Comparative Example 5 | Comparative Compound 4 | 4.9 | Blue | 270 |

### Example 7 (production of organic EL device)

An organic EL device was produced in the same manner as in Example 1 except that the following Arylamine Compound D2 was used instead of the Amine Compound D1 having a styryl group. Me represents a methyl group.
The luminous efficiency of the resultant organic EL device was measured, and the result showed 5.2 cd/A. The luminescent color of the device was blue. Further, the half lifetime of light emission in DC constant current driving at an initial luminance of 5,000 nits and room temperature was measured. The result was 400 hours.

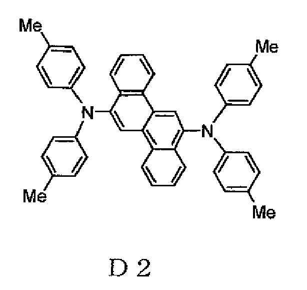

### Comparative Example 3

An organic EL device was produced in the same manner as in Example 7 except that the above Comparative Compound 1 was used as a hole transporting material instead of Compound H1.
The luminous efficiency of the resultant organic EL device was measured, and the result showed 4. 9 cd/A. The luminescent color of the device was blue. Further, the half lifetime of light emission in DC constant current driving at an initial luminance of 5,000 nits and room temperature was measured. The result was 260 hours.

### Comparative Examples 4 and 5

An organic EL device was produced in the same manner as in Example 1 except that the above Comparative Compounds 3 and 4 were used in Comparative Examples 4 and 5, respectively, each as a hole transporting material instead of Compound H1.
The luminous efficiency of the resultant organic EL device was measured, and the luminescent color of the device was observed. Further, the half lifetime of light emission in DC constant current driving at an initial luminance of 5,000 nits and room temperature was measured. Table 1 shows the results thereof.

### Industrial Applicability

As described above in detail, the aromatic amine derivative of the present invention has a small interaction between molecules because of steric hindrance thereof, which contributes to suppress crystallization of the aromatic amine derivative and improvement of yield in the organic EL device production. Further, the aromatic amine derivative can be deposited by vapor at a low sublimation temperature, so decomposition of a molecule therein is suppressed at the time of vapor deposition. As a result, an organic EL device having a long lifetime can be obtained.

## Claims

1. An aromatic amine derivative represented by the following general formula (1): 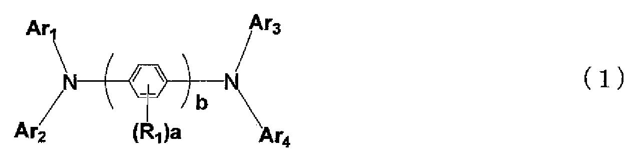 where: R₁ represents a substituted or unsubstituted aryl group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 2 to 50 carbon atoms, an amino group substituted by a substituted or unsubstituted aryl group having 5 to 50 ring atoms, a halogen group, a cyano group, a nitro group, a hydroxyl group, or a carboxyl group;
a represents an integer of 0 to 4, and when a represents 2 or more, a plurality of R₁'s may be bonded to each other to form a saturated or unsaturated, five- or six-membered cyclic structure which may be substituted;
b represents an integer of 1 to 3, and when a represents 1 or more and b represents 2 or more, a plurality of R₁'s may be bonded to each other to forma saturated or unsaturated, five- or six-membered cyclic structure which may be substituted; and
at least one of Ar₁ to Ar₄ is a group represented by the following general formula (2): 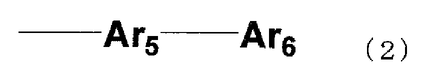 where:
Ar₅ represents a substituted or unsubstituted fused aromatic ring group having 10 to 20 ring carbon atoms; and
Ar₆ represents a substituted or unsubstituted aryl group having 6 to 50 ring carbon atoms or a substituted or unsubstituted aromatic heterocyclic group having 5 to 50 ring carbon atoms,
where remaining groups of Ar₁ to Ar₄, none of which is represented by the general formula (2), each independently represent a substituted or unsubstituted aryl group having 6 to 50 ring carbon atoms or a substituted or unsubstituted aromatic heterocyclic group having 5 to 50 ring carbon atoms.

2. An aromatic amine derivative according to claim 1, wherein each of Ar₁ and Ar₂ in the general formula (1) is a group represented by the general formula (2).

3. An aromatic amine derivative according to claim 1, wherein each of Ar₁ and Ar₃ in the general formula (1) is a group represented by the general formula (2).

4. An aromatic amine derivative according to claim 1, wherein Ar₁ in the general formula (1) is a group represented by the general formula (2).

5. An aromatic amine derivative according to claim 1, wherein b in the general formula (1) represents 2.

6. An aromatic amine derivative according to claim 1, wherein Ar₅ in the general formula (2) represents a divalent residue of naphthalene, phenanthrene, or pyrene.

7. An aromatic amine derivative according to claim 1, wherein Ar₅ in the general formula (2) is a group represented by the following general formula (3): 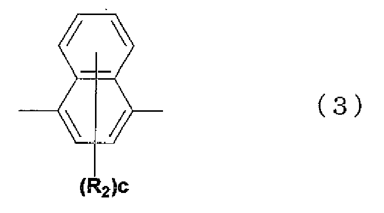 where:
R₂ is selected from the same group as that of R₁ in the general formula (1); and
c represents an integer of 0 to 6.

8. An aromatic amine derivative according to claim 1, wherein Ar₆ in the general formula (2) represents a phenyl group, a biphenyl group, or a naphthyl group.

9. An aromatic amine derivative according to claim 1, wherein Ar₂ in the general formula (1) is a group represented by the following general formula (4): 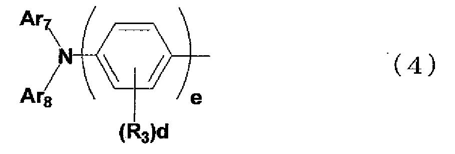 where:
R₃ is selected from the same group as that of R₁ in the general formula (1);
d represents an integer of 0 to 4, and when d represents 2 or more, a plurality of R₃'s may be bonded to each other to form a saturated or unsaturated, five- or six-membered cyclic structure which may be substituted;
e represents an integer of 1 to 3, and when d represents 1 or more and e represents 2 or more, a plurality of R₃'s may be bonded to each other to forma saturated or unsaturated, five- or six-membered cyclic structure which may be substituted; and
Ar₇ and Ar₈ each independently is a group represented by the general formula (2), a substituted or unsubstituted aryl group having 6 to 50 ring carbon atoms, or a substituted or unsubstituted aromatic heterocyclic group having 5 to 50 ring carbon atoms.

10. An aromatic amine derivative according to claim 1, wherein Ar₂ and Ar₄ in the general formula (1) each independently is a group represented by the general formula (4).

11. An aromatic amine compound according to any one of claims 1 to 10, comprising a material for an organic electroluminescent device.

12. An aromatic amine compound according to any one of claims 1 to 10, further comprising a hole transporting material for an organic electroluminescent device.

13. An organic electroluminescent device, comprising an organic thin film layer formed of one or a plurality of layers including at least a light emitting layer interposed between a cathode and an anode, wherein at least one layer of the organic thin film layer contains the aromatic amine derivative according to any one of claims 1 to 10 alone or as a component of a mixture.

14. An organic electroluminescent device, wherein the aromatic amine derivative according to any one of claims 1 to 10 is contained in a hole transporting layer.

15. An organic electroluminescent device according to claim 13, wherein:
the organic thin film layer comprises a hole transporting layer, and an electron transporting layer or an electorn injecting layer;
the hole transporting layer contains the aromatic amine derivative; and
the electron transporting layer or the electorn injecting layer contains a nitrogen-containing heterocyclic compound.

16. An organic electroluminescent device according to claim 13, which emits blue-based light.

17. An organic electroluminescent device according to claim 16, further comprising a styrylamine and/or an arylamine in a light emitting layer.
